(19) Europäisches Patentamt
European Patent Office
Office européen des brevets

(11) EP 2 287 320 B1

(12) EUROPEAN PATENT SPECIFICATION

(45) Date of publication and mention of the grant of the patent:
**01.10.2014 Bulletin 2014/40**

(21) Application number: **10010298.7**

(22) Date of filing: **16.01.2003**

(51) Int Cl.:
*C12N 15/57* (2006.01)
*C12N 15/74* (2006.01)
*C12N 9/54* (2006.01)
*C12N 1/21* (2006.01)
*C11D 3/386* (2006.01)

(54) **Multiply-substituted protease variants**

Mehrfach substituierte Proteasevarianten

Variantes multiples de protéase substituée

(84) Designated Contracting States:
**AT BE BG CH CY CZ DE DK EE ES FI FR GB GR HU IE IT LI LU MC NL PT SE SI SK TR**

(30) Priority: **16.01.2002 US 350221 P**

(43) Date of publication of application:
**23.02.2011 Bulletin 2011/08**

(62) Document number(s) of the earlier application(s) in accordance with Art. 76 EPC:
**03731959.7 / 1 530 631**

(73) Proprietor: **Danisco US Inc.**
**Palo Alto, CA 94304-1013 (US)**

(72) Inventor: **Poulose, Ayrookaran J.**
**Belmont, CA 94002 (US)**

(74) Representative: **Forrest, Graham Robert et al**
**Mewburn Ellis LLP**
**33 Gutter Lane**
**London**
**EC2V 8AS (GB)**

(56) References cited:
- **DATABASE Geneseq [Online] EBI, Hinxton, Cambridgeshire, U.K.; 23 May 1991 (1991-05-23), Wilson CR, et al: "Pre-pro alkaline protease enzyme", XP002613313, Database accession no. AAR11190 & WO 91/02792 A1 (HENKEL RESEARCH CORP [US]) 7 March 1991 (1991-03-07)**
- **DATABASE Geneseq [Online] EBI, Hinxton, Cambridgeshire, U.K.; 1 November 1989 (1989-11-01), Hastrup S, et al.,: "Subtilisin 309 from Bacillus lentus", XP002613314, Database accession no. AAP90374 & WO 89/06279 A1 (NOVO INDUSTRI AS [DK]) 13 July 1989 (1989-07-13)**
- **DATABASE Geneseq [Online] EBI, Hinxton, Cambridgeshire, U.K.; 1 November 1989 (1989-11-01), Hastrup S, et al.,: "Subtilisin BPN' from Bacillus spp.", XP002613315, Database accession no. AAP90376**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 287 320 B1

**Description**

## Background of the Invention

Serine proteases are a subgroup of carbonyl hydrolases. They comprise a diverse class of enzymes having a wide range of specificities and biological functions. Stroud, R. Sci. Amer., 131:74-88. Despite their functional diversity, the catalytic machinery of serine proteases has been approached by at least two genetically distinct families of enzymes: 1) the subtilisins and 2) the mammalian chymotrypsin-related and homologous bacterial serine proteases (e.g., trypsin and *S. gresius* trypsin). These two families of serine proteases show remarkably similar mechanisms of catalysis. Kraut, J. (1977), Annu. Rev. Biochem., 46:331-358. Furthermore, although the primary structure is unrelated, the tertiary structure of these two enzyme families bring together a conserved catalytic triad of amino acids consisting of serine, histidine and aspartate.

Subtilisins are serine proteases (approx. MW 27,500) which are secreted in large amounts from a wide variety of *Bacillus* species and other microorganisms. The protein sequence of subtilisin has been determined from at least nine different species of *Bacillus.* Markland, F.S., et al. (1983), Hoppe-Seyler's Z. Physiol. Chem., 364:1537-1540. The three-dimensional crystallographic structure of subtilisins from *Bacillus amyloliquefaciens, Bacillus licheniforimis* and several natural variants of *B. lentus* have been reported. These studies indicate that although subtilisin is genetically unrelated to the mammalian serine proteases, it has a similar active site structure. The x-ray crystal structures of subtilisin containing covalently bound peptide inhibitors (Robertus, J.D., et al. (1972), Biochemistry, 11:2439-2449) or product complexes (Robertus, J.D., et al. (1976), J. Biol. Chem., 251:1097-1103) have also provided information regarding the active site and putative substrate binding cleft of subtilisin. In addition, a large number of kinetic and chemical modification studies have been reported for subtilisin; Svendsen, B. (1976), Carlsberg Res. Commun., 41:237-291; Markland, F.S. Id.) as well as at least one report wherein the side chain of methionine at residue 222 of subtilisin was converted by hydrogen peroxide to methionine-sulfoxide (Stauffer, D.C., et al. (1965), J. Biol. Chem., 244:5333-5338) and extensive site-specific mutagenesis has been carried out (Wells and Estell (1988) TIBS 13:291-297)

## Summary of the Invention

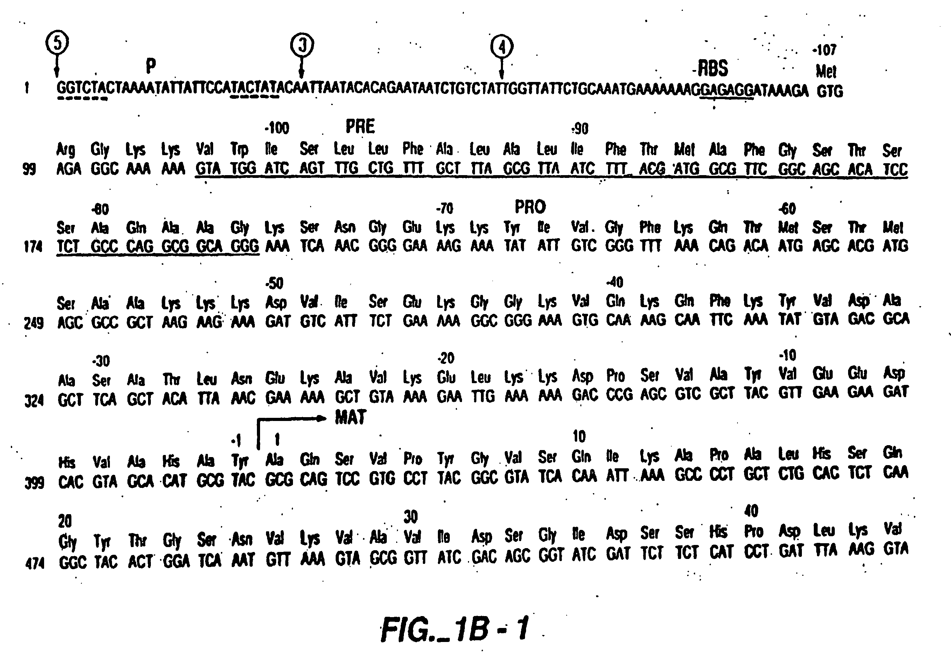

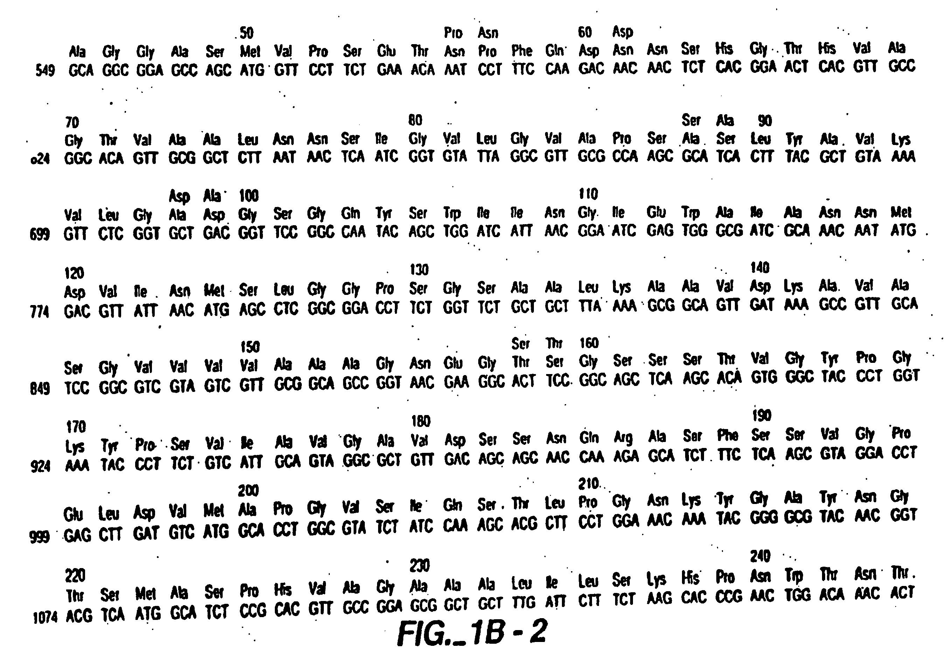

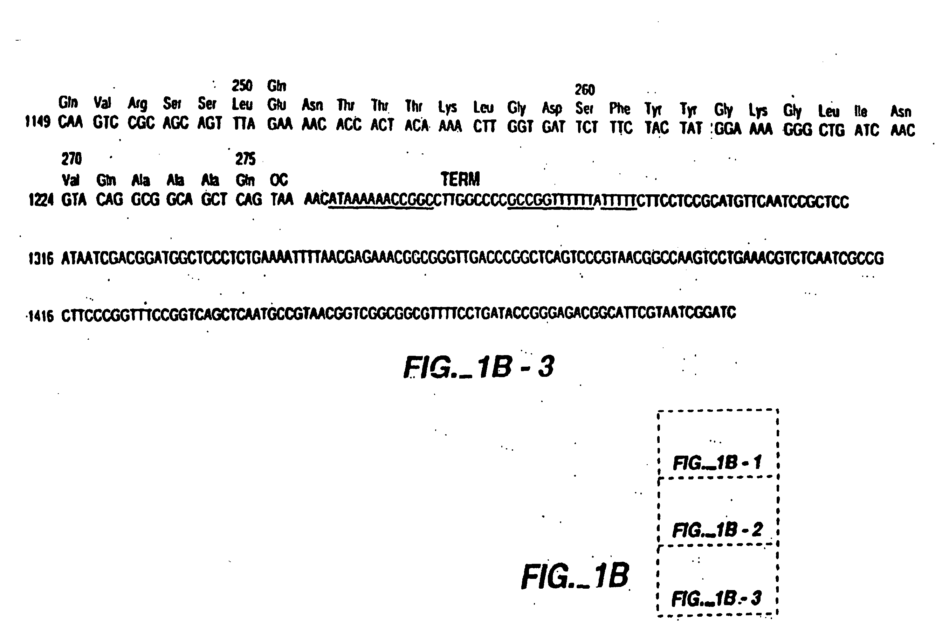

The invention provides a variant *Bacillus* subtilisin protease comprising an amino acid sequence having a substitution at a residue position equivalent to residue position 148 of *Bacillus amyloliquefaciens* subtilisin having the sequence set forth in SEQ ID NO: 2 (Figure 1B), wherein said variant has improved wash performance and/or thermostability as compared to the precursor subtilisin protease from which the variant is derived.

Also provided is a method of improving the wash performance or thermostability of a precursor *Bacillus* subtilisin protease, comprising making a substitution in said precursor subtilisin protease at a residue position equivalent to residue position 148 of *Bacillus amyloliquefaciens* subtilisin having the sequence set forth in SEQ ID NO: 2 (Figure 1B) to produce a variant subtilisin protease, wherein said variant subtilisin protease has improved wash performance and/or thermostability as compared to said precursor protease.

The method may comprise providing a DNA sequence encoding said variant subtilisin protease and expressing said variant subtilisin protease from said DNA sequence.

The method may comprise providing a precursor DNA sequence encoding said precursor subtilisin protease and modifying said precursor DNA sequence to obtain a variant DNA sequence encoding said variant subtilisin protease.

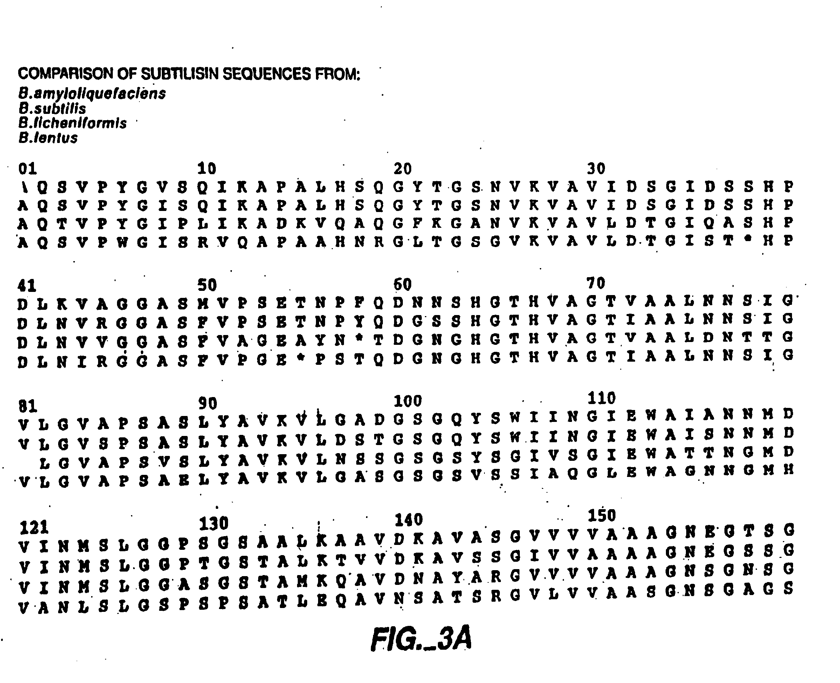

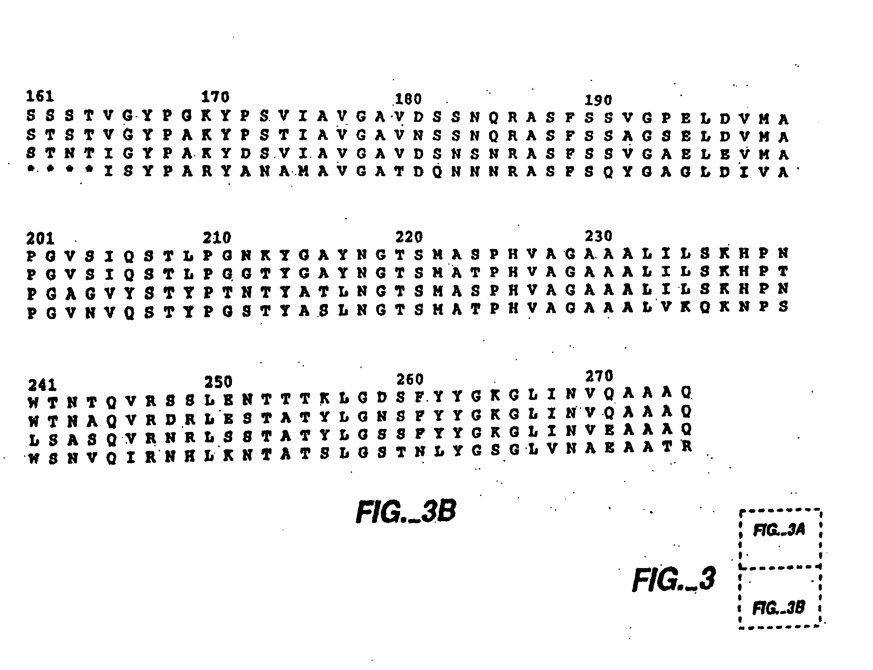

The substitution may be equivalent to the substitution L148G or L148W in *Bacillus lentus* subtilisin having the sequence set forth in SEQ ID NO: 6 (Figure 3).

The precursor subtilisin may be *Bacillus amyloliquefaciens* subtilisin, *Bacillus lentus* subtilisin, or subtilisin 309.

Also provided is a DNA encoding a variant subtilisin protease of the invention, an expression vector encoding such a DNA, and a host cell transformed with such an expression vector.

Also provided is a cleaning composition comprising the variant subtilisin protease of the invention.

## BRIEF DESCRIPTION OF THE DRAWINGS

Figs. 1 A-C depict the DNA (SEQ ID NO:1) and amino acid sequences (SEQ ID NO:2) for *Bacillus amyloliquefaciens* subtilisin and a partial restriction map of this gene.

Fig. 2 depicts the conserved amino acid residues among subtilisins from *Bacillus amyloliquefaciens* (BPN)' and *Bacillus lentus* (wild-type).

Figs. 3A and 3B depict the amino acid sequence of four subtilisins. The top line represents the amino acid sequence of subtilisin from *Bacillus amyloliquefaciens* subtilisin (also sometimes referred to as subtilisin BPN') (SEQ ID NO:3).

The second line depicts the amino acid sequence of subtilisin from *Bacillus subtilis* (SEQ ID NO:4). The third line depicts the amino acid sequence of subtilisin from *B. licheniformis* (SEQ ID NO:5). The fourth line depicts the amino acid sequence of subtilisin from *Bacillus lentus* (also referred to as subtilisin 309 in PCT WO89/06276) (SEQ ID NO:6). The symbol * denotes the absence of specific amino acid residues as compared to subtilisin BPN'.

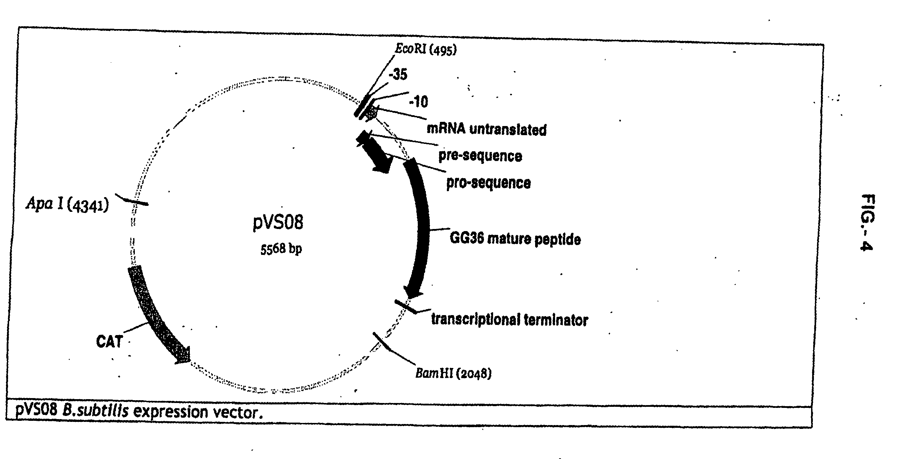

Fig 4 depicts the pVS08 B. subtilis expression vector.

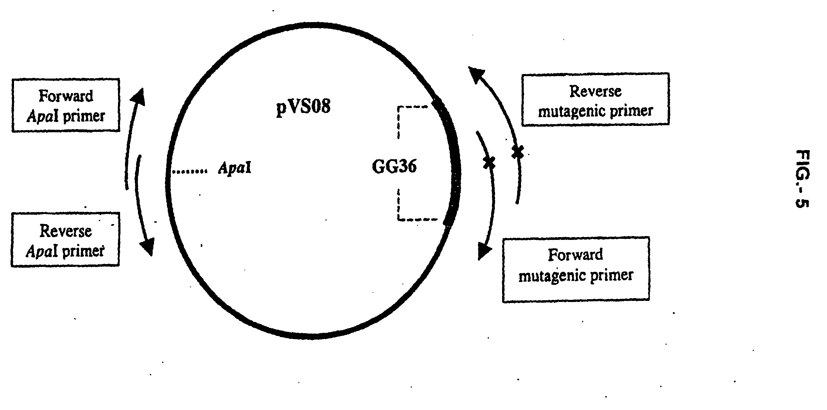

Fig. 5 depicts the orientation of the forward ApaI primer, the reverse ApaI primer, the reverse mutagenic primer, and the forward mutagenic primer.

## Detailed Description of the Invention

Proteases are carbonyl hydrolases which generally act to cleave peptide bonds of proteins or peptides. As used herein, "protease" means a naturally-occurring protease or a recombinant protease. Naturally-occurring hydrolase, peptidylamino acid hydrolase, acylamino hydrolase, serine carboxypeptidase, metallocarboxypeptidase, thiol proteinase, carboxyl-proteinase and metalloproteinase. Serine, metallo, thiol and acid proteases are included, as well as endo and exo-proteases.

The present invention includes protease enzymes which are non-naturally occurring carbonyl hydrolase variants (protease variants) having a different proteolytic activity, stability, substrate specificity, pH profile and/or performance characteristic as compared to the precursor carbonyl hydrolase from which the amino acid sequence of the variant is derived. Specifically, such protease variants have an amino acid sequence not found in nature, which is derived by substitution of a plurality of amino acid residues of a precursor protease with different amino acids. The precursor protease may be a naturally-occurring protease or a recombinant protease.

The protease variants useful herein encompass the substitution of any of the nineteen naturally occurring L-amino acids at the designated amino acid residue positions. Such substitutions can be made in any precursor subtilisin (procaryotic, eucaryotic, mammalian, etc.). Throughout this application reference is made to various amino acids by way of common one - and three-letter codes. Such codes are identified in Dale, M.W. (1989), Molecular Genetics of Bacteria, John Wiley & Sons, Ltd., Appendix B.

The protease variants useful herein are preferably derived from a *Bacillus* subtilisin. More preferably, the protease variants are derived from *Bacillus amyloliquefaciens, Bacillus lentus* subtilisin and/or subtilisin 309.

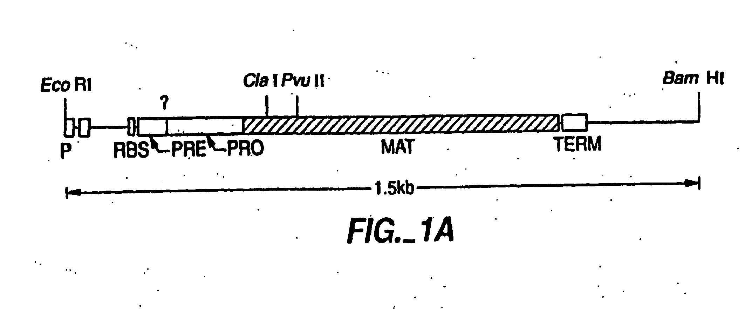

Subtilisins are bacterial or fungal proteases which generally act to cleave peptide bonds of proteins or peptides. As used herein, "subtilisin" means a naturally-occurring subtilisin or a recombinant subtilisin. A series of naturally-occurring subtilisins is known to be produced and often secreted by various microbial species. Amino acid sequences of the members of this series are not entirely homologous. However, the subtilisins in this series exhibit the same or similar type of proteolytic activity. This class of serine proteases shares a common amino acid sequence defining a catalytic triad which distinguishes them from the chymotrypsin related class of serine proteases. The subtilisins and chymotrypsin related serine proteases both have a catalytic triad comprising aspartate, histidine and serine. In the subtilisin related proteases the relative order of these amino acids, reading from the amino to carboxy terminus, is aspartate-histidine-serine. In the chymotrypsin related proteases, the relative order, however, is histidine-aspartate-serine. Thus, subtilisin herein refers to a serine protease having the catalytic triad of subtilisin related proteases. Examples include but are not limited to the subtilisins identified in Fig. 3 herein. Generally and for purposes of the present invention, numbering of the amino acids in proteases corresponds to the numbers assigned to the mature *Bacillus amyloliquefaciens* subtilisin sequence presented in Figure1.

"Recombinant subtilisin" or "recombinant protease" refer to a subtilisin or protease in which the DNA sequence encoding the subtilisin or protease is modified to produce a variant (or mutant) DNA sequence which encodes the substitution, deletion or insertion of one or more amino acids in the naturally-occurring amino acid sequence. Suitable methods to produce such modification, and which may be combined with those disclosed herein, include those disclosed in US Patent RE 34,606, US Patent 5,204,015 and US Patent 5,185,258, U.S. Patent 5,700,676, U.S. Patent 5,801,038, and U.S. Patent 5,763,257.

"Non-human subtilisins" and the DNA encoding them may be obtained from many procaryotic and eucaryotic organisms. Suitable examples of procaryotic organisms include gram negative organisms such as *E. coli* or *Pseudomonas* and gram positive bacteria such as *Micrococcus* or *Bacillus.* Examples of eucaryotic organisms from which subtilisin and their genes may be obtained include yeast such as *Saccharomyces cerevisiae,* fungi such as *Aspergillus* sp.

A "protease variant" has an amino acid sequence which is derived from the amino acid sequence of a "precursor protease". The precursor proteases include naturally-occurring proteases and recombinant proteases. The amino acid sequence of the protease variant is "derived" from the precursor protease amino acid sequence by the substitution, deletion or insertion of one or more amino acids of the precursor amino acid sequence. Such modification is of the "precursor DNA sequence" which encodes the amino acid sequence of the precursor protease rather than manipulation of the precursor protease enzyme *per se.* Suitable methods for such manipulation of the precursor DNA sequence include methods disclosed herein, as well as methods known to those skilled in the art (see, for example, EP 0 328299, WO89/06279 and the US patents and applications already referenced herein).

Substitution of an amino acid at a residue position equivalent to residue position 148 of *Bacillus amyloliquefaciens* subtilisin is identified herein as providing improved wash performance under Japanese wash conditions.

Substitution of an amino acid at a residue position equivalent to residue position 148 of *Bacillus amyloliquefaciens* subtilisin is identified herein as providing improved thermostability under European wash conditions.

Amino acid position numbers used herein refer to those assigned to the mature *Bacillus amyloliquefaciens* subtilisin sequence presented in Fig. 1 (SEQ ID NO: 2). The invention, however, is not limited to the mutation of this particular subtilisin but extends to precursor proteases containing amino acid residues at positions which are "equivalent" to the particular identified residues in *Bacillus amyloliquefaciens* subtilisin. In a preferred embodiment of the present invention, the precursor protease is *Bacillus lentus* subtilisin and the substitutions are made at the equivalent amino acid residue positions in B. *lentus* corresponding to those listed above.

A residue (amino acid) position of a precursor protease is equivalent to a residue of *Bacillus amyloliquefaciens* subtilisin if it is either homologous (i.e., corresponding in position in either primary or tertiary structure) or analogous to a specific residue or portion of that residue in *Bacillus amyloliquefaciens* subtilisin (i.e., having the same or similar functional capacity to combine, react, or interact chemically).

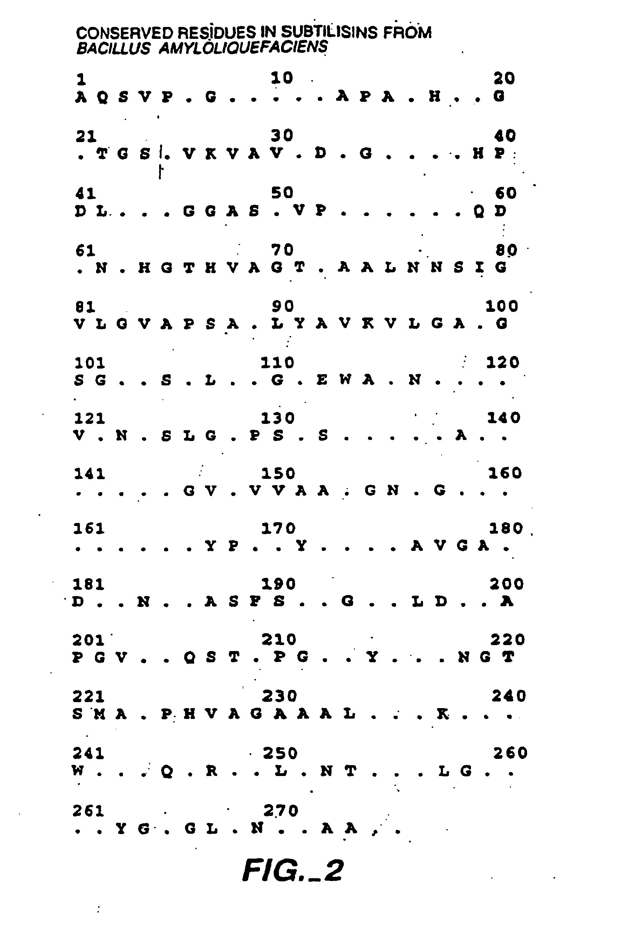

In order to establish homology to primary structure, the amino acid sequence of a precursor protease is directly compared to the *Bacillus amyloliquefaciens* subtilisin primary sequence and particularly to a set of residues known to be invariant in subtilisins for which sequence is known. For example, Fig. 2 herein shows the conserved residues as between *B. amyloliquefaciens* subtilisin and *B. lentus* subtilisin. After aligning the conserved residues, allowing for necessary insertions and deletions in order to maintain alignment (i.e., avoiding the elimination of conserved residues through arbitrary deletion and insertion), the residues equivalent to particular amino acids in the primary sequence of *Bacillus amyloliquefaciens* subtilisin are defined. Alignment of conserved residues preferably should conserve 100% of such residues. However, alignment of greater than 98%, 95%, 90%, 85%, 80% , 75% 70%, 50% or at least 45% of conserved residues is also adequate to define equivalent residues. Conservation of the catalytic triad, Asp32/His64/Ser221 should be maintained. Siezen et al. (1991) Protein Eng. 4(7):719-737 shows the alignment of a large number of serine proteases. Siezen et al. refer to the grouping as subtilases or subtilisin-like serine proteases.

For example, in Fig. 3, the amino acid sequence of subtilisin from *Bacillus amyloliquefaciens, Bacillus subtilis, Bacillus licheniformis (carlsbergensis)* and *Bacillus lentus* are aligned to provide the maximum amount of homology between amino acid sequences. A comparison of these sequences shows that there are a number of conserved residues contained in each sequence. These conserved residues (as between BPN' and *B. lentus)* are identified in Fig. 2.

These conserved residues, thus, may be used to define the corresponding equivalent amino acid residues of *Bacillus amyloliquefaciens* subtilisin in other subtilisins such as subtilisin from *Bacillus lentus* (PCT Publication No. WO89/06279 published July 13, 1989), the preferred protease precursor enzyme herein, or the subtilisin referred to as PB92 (EP 0 328 299), which is highly homologous to the preferred *Bacillus lentus* subtilisin. The amino acid sequences of certain of these subtilisins are aligned in Figs. 3A and 3B with the sequence of *Bacillus amyloliquefaciens* subtilisin to produce the maximum homology of conserved residues. As can be seen, there are a number of deletions in the sequence of *Bacillus lentus* as compared to *Bacillus amyloliquefaciens* subtilisin. Thus, for example, the equivalent amino acid for Val165 in *Bacillus amyloliquefaciens* subtilisin in the other subtilisins is isoleucine for *B. lentus* and *B. licheniformis.*

"Equivalent residues" may also be defined by determining homology at the level of tertiary structure for a precursor protease whose tertiary structure has been determined by x-ray crystallography. Equivalent residues are defined as those for which the atomic coordinates of two or more of the main chain atoms of a particular amino acid residue of the precursor protease and *Bacillus amyloliquefaciens* subtilisin (N on N, CA on CA, C on C and O on O) are within 0.13nm and preferably 0.1 nm after alignment. Alignment is achieved after the best model has been oriented and positioned to give the maximum overlap of atomic coordinates of non-hydrogen protein atoms of the protease in question to the *Bacillus amyloliquefaciens* subtilisin. The best model is the crystallographic model giving the lowest R factor for experimental diffraction data at the highest resolution available.

$$R\ factor = \frac{\Sigma_h |Fo(h)| - |Fc(h)|}{\Sigma_h |Fo(h)|}$$

Equivalent residues which are functionally similar to a specific residue of *Bacillus amyloliquefaciens* subtilisin are defined as those amino acids of the precursor protease which may adopt a conformation such that they either alter, modify or contribute to protein structure, substrate binding or catalysis in a manner defined and attributed to a specific residue of the *Bacillus amyloliquefaciens* subtilisin. Further, they are those residues of the precursor protease (for which a tertiary structure has been obtained by x-ray crystallography) which occupy an analogous position to the extent that, although the main chain atoms of the given residue may not satisfy the criteria of equivalence on the basis of occupying a homologous position, the atomic coordinates of at least two of the side chain atoms of the residue lie with 0.13nm of the corresponding side chain atoms of *Bacillus amyloliquefaciens* subtilisin. The coordinates of the three dimensional structure of *Bacillus amyloliquefaciens* subtilisin are set forth in EPO Publication No. 0 251 446 (equivalent to US Patent 5,182,204, the disclosure of which is incorporated herein by reference) and can be used as outlined above to determine equivalent residues on the level of tertiary structure.

Some of the residues identified for substitution are conserved residues whereas others are not. In the case of residues which are not conserved, the substitution of one or more amino acids is limited to substitutions which produce a variant which has an amino acid sequence that does not correspond to one found in nature. In the case of conserved residues, such substitutions should not result in a naturally-occurring sequence. The protease variants of the present invention include the mature forms of protease variants, as well as the pro- and prepro-forms of such protease variants. The prepro-forms are the preferred construction since this facilitates the expression, secretion and maturation of the protease variants.

"Prosequence" refers to a sequence of amino acids bound to the N-terminal portion of the mature form of a protease which when removed results in the appearance of the "mature" form of the protease. Many proteolytic enzymes are found in nature as translational proenzyme products and, in the absence of post-translational processing, are expressed in this fashion. A preferred prosequence for producing protease variants is the putative prosequence of *Bacillus amyloliquefaciens* subtilisin, although other protease prosequences may be used.

A "signal sequence" or "presequence" refers to any sequence of amino acids bound to the N-terminal portion of a protease or to the N-terminal portion of a proprotease which may participate in the secretion of the mature or pro forms of the protease. This definition of signal sequence is a functional one, meant to include all those amino acid sequences encoded by the N-terminal portion of the protease gene which participate in the effectuation of the secretion of protease under native conditions. The present invention utilizes such sequences to effect the secretion of the protease variants as defined herein. One possible signal sequence comprises the first seven amino acid residues of the signal sequence from *Bacillus subtilis* subtilisin fused to the remainder of the signal sequence of the subtilisin from *Bacillus lentus* (ATCC 21536).

A "prepro" form of a protease variant consists of the mature form of the protease having a prosequence operably linked to the amino terminus of the protease and a "pre" or "signal" sequence operably linked to the amino terminus of the prosequence.

"Expression vector" refers to a DNA construct containing a DNA sequence which is operably linked to a suitable control sequence capable of effecting the expression of said DNA in a suitable host. Such control sequences include a promoter to effect transcription, an Optional operator sequence to control such transcription, a sequence encoding suitable mRNA ribosome binding sites and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. In the present specification, "plasmid" and "vector" are sometimes used interchangeably as the plasmid is the most commonly used form of vector at present. However, the invention is intended to include such other forms of expression vectors which serve equivalent functions and which are, or become, known in the art.

The "host cells" used in the present invention generally are procaryotic or eucaryotic hosts which preferably have been manipulated by the methods disclosed in US Patent RE 34,606 and/or 5,441,882 to render them incapable of secreting enzymatically active endoprotease. A host cell useful for expressing protease is the *Bacillus* strain BG2036 which is deficient in enzymatically active neutral protease and alkaline protease (subtilisin). The construction of strain BG2036 is described in detail in US Patent 5,264,366. Other host cells for expressing protease include *Bacillus subtilis* 1168 (also described in US Patent RE 34,606; US Patent 5,264,366; and 5,441,882, the disclosure of which are incorporated herein by reference), as well as any suitable *Bacillus* strain such as *B. licheniformis, B. lentus,* etc. A particularly useful host cell is the Bacillus strain BG2864. The construction of strain BG2864 is described in detail in D. Naki, C. Paech, G. Ganshaw, V. Schellenberger. Appl Microbiol Biotechnol (1998) 49:290-294.

Host cells are transformed or transfected with vectors constructed using recombinant DNA techniques. Such transformed host cells are capable of either replicating vectors encoding the protease variants or expressing the desired protease variant. In the case of vectors which encode the pre- or prepro-form of the protease variant, such variants, when expressed, are typically secreted from the host cell into the host cell medium.

"Operably linked, "when describing the relationship between two DNA regions, simply means that they are functionally related to each other. For example, a presequence is operably linked to a peptide if it functions as a signal sequence, participating in the secretion of the mature form of the protein most probably involving cleavage of the signal sequence. A promoter is operably linked to a coding sequence if it controls the transcription of the sequence; a ribosome binding site is operably linked to a coding sequence if it is positioned so as to permit translation.

The genes encoding the naturally-occurring precursor protease may be obtained in accord with the general methods known to those skilled in the art. The methods generally comprise synthesizing labeled probes having putative sequences encoding regions of the protease of interest, preparing genomic libraries from organisms expressing the protease, and screening the libraries for the gene of interest by hybridization to the probes. Positively hybridizing clones are then mapped and sequenced.

The cloned protease is then used to transform a host cell in order to express the protease. The protease gene is then ligated into a high copy number plasmid. This plasmid replicates in hosts in the sense that it contains the well-known elements necessary for plasmid replication: a promoter operably linked to the gene in question (which may be supplied as the gene's own homologous promoter if it is recognized, i.e., transcribed, by the host), a transcription termination and polyadenylation region (necessary for stability of the mRNA transcribed by the host from the protease gene in certain eucaryotic host cells) which is exogenous or is supplied by the endogenous terminator region of the protease gene and, desirably, a selection gene such as an antibiotic resistance gene that enables continuous cultural maintenance of plasmid-infected host cells by growth in antibiotic-containing media. High copy number plasmids also contain an origin of replication for the host, thereby enabling large numbers of plasmids to be generated in the cytoplasm without chromosomal limitations. However, it is within the scope herein to integrate multiple copies of the protease gene into host genome. This is facilitated by procaryotic and eucaryotic organisms which are particularly susceptible to homologous recombination.

The gene can be a natural *B. lentus* gene. Alternatively, a synthetic gene encoding a naturally-occurring or mutant precursor protease may be produced. In such an approach, the DNA and/or amino acid sequence of the precursor protease is determined. Multiple, overlapping synthetic single-stranded DNA fragments are thereafter synthesized, which upon hybridization and ligation produce a synthetic DNA encoding the precursor protease. An example of synthetic gene construction is set forth in Example 3 of US Patent 5,204,015.

Once the naturally-occurring or synthetic precursor protease gene has been cloned, a number of modifications are undertaken to enhance the use of the gene beyond synthesis of the naturally-occurring precursor protease. Such modifications include the production of recombinant proteases as disclosed in US Patent RE 34,606 and EPO Publication No. 0 251 446 and the production of protease variants described herein.

The following cassette mutagenesis method may be used to facilitate the construction of the protease variants of the present invention, although other methods may be used. First, the naturally-occurring gene encoding the protease is obtained and sequenced in whole or in part. Then the sequence is scanned for a point at which it is desired to make a mutation (deletion, insertion or substitution) of one or more amino acids in the encoded enzyme. The sequences flanking this point are evaluated for the presence of restriction sites for replacing a short segment of the gene with an oligonucleotide pool which when expressed will encode various mutants. Such restriction sites are preferably unique sites within the protease gene so as to facilitate the replacement of the gene segment. However, any convenient restriction site which is not overly redundant in the protease gene may be used, provided the gene fragments generated by restriction digestion can be reassembled in proper sequence. If restriction sites are not present at locations within a convenient distance from the selected point (from 10 to 15 nucleotides), such sites are generated by substituting nucleotides in the gene in such a fashion that neither the reading frame nor the amino acids encoded are changed in the final construction. Mutation of the gene in order to change its sequence to conform to the desired sequence is accomplished by M13 primer extension in accord with generally known methods. The task of locating suitable flanking regions and evaluating the needed changes to arrive at two convenient restriction site sequences is made routine by the redundancy of the genetic code, a restriction enzyme map of the gene and the large number of different restriction enzymes. Note that if a convenient flanking restriction site is available, the above method need be used only in connection with the flanking region which does not contain a site.

Once the naturally-occurring DNA or synthetic DNA is cloned, the restriction sites flanking the positions to be mutated are digested with the cognate restriction enzymes and a plurality of end termini-complementary oligonucleotide cassettes are ligated into the gene. The mutagenesis is simplified by this method because all of the oligonucleotides can be synthesized so as to have the same restriction sites, and no synthetic linkers are necessary to create the restriction sites.

As used herein, proteolytic activity is defined as the rate of hydrolysis of peptide bonds per milligram of active enzyme. Many well known procedures exist for measuring proteolytic activity (K. M. Kalisz, "Microbial Proteinases," Advances in Biochemical Engineering/Biotechnology, A. Fiechter ed., 1988). In addition to or as an alternative to modified proteolytic activity, the variant enzymes of the present invention may have other modified properties such as $K_m$, $k_{cat}$, $k_{cat}/K_m$ ratio and/or modified substrate specificity and/or modified pH activity profile. These enzymes can be tailored for the particular substrate which is anticipated to be present, for example, in the preparation of peptides or for hydrolytic processes such as laundry uses.

Stability, for example thermostability, is an aspect which could be accomplished by the protease variant described in the examples. The stability may be enhanced or diminished as is desired for various uses. Enhanced stability could be effected by substitution one or more residues identified in the present application and, optionally, substituting another amino acid residue not one of the same. Thermostability is maintaining enzymatic acitivty over time at a given temperature. An improved thermostability involves the maintenance of a greater amount of enzymatic acitivity by the variant as compared to the precursor protease. For example, an increased level of enzymatic activity of the variant as compared to the precursor at a given temperature, typically the operation temperature of as measured.

There is a variety of wash conditions including varying detergent formulations, wash water volume, wash water temperature and length of wash time that a protease variant might be exposed to. For example, detergent formulations used in different areas have different concentrations of their relevant components present in the wash water. For example, a European detergent typically has about 3000-8000 ppm of detergent components in the wash water while a Japanese detergent typically has less than 800, for example 667 ppm of detergent components in the wash water. In North America, particularly the United States, a detergent typically has about 800 to 2000, for example 975 ppm of detergent components present in the wash water.

A low detergent concentration system includes detergents where less than about 800 ppm of detergent components are present in the wash water. Japanese detergents are typically considered low detergent concentration system as they have approximately 667 ppm of detergent components present in the wash water.

A medium detergent concentration includes detergents where between about 800 ppm and about 2000ppm of detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of detergent components are present in the wash water. European detergents are generally considered to be high detergent concentration systems as they have approximately 3000-8000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent composition ("low detergent concentration geographies"), for example about 667 ppm in Japan, to between about 800 ppm to about 2000 ppm ("medium detergent concentration geographies"), for example about 975 ppm in U.S. and about 1500 ppm in Brazil, to greater than about 2000 ppm ("high detergent concentration geographies"), for example about 3000 ppm to about 8000 ppm in Europe and about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan can be between 10 and 30 degrees centigrade, for example about 20 degrees C, whereas the temperature of wash water in Europe is typically between 30 and 50 degrees centigrade, for example about 40 degrees C.

As a further example, different geographies may have different water hardness. Water hardness is typically described as grains per gallon mixed $Ca^{2+}/Mg^{2+}$. Hardness is a measure of the amount of calcium ($Ca^{2+}$) and magnesium ($Mg^{2+}$) in the water. Most water in the United States is hard, but the degree of hardness varies. Moderately hard (60-120 ppm) to hard (121-181 ppm) water has 60 to 181 parts per million [parts per million converted to grains per U.S. gallon is ppm # divided by 17.1 equals grains per gallon] of hardness minerals.

| Water | Grains per gallon | Parts per million |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than 10.5 (for example 10.5-20.0) grains per gallon mixed $Ca^{2+}/Mg^{2+}$, for example about 15 grains per gallon mixed $Ca^{2+}/Mg^{2+}$. North American water hardness is typically greater than Japanese water hardness, but less than European water hardness. For example, North American water hardness can be between 3 to10 grains, 3-8 grains or about 6 grains. Japanese water hardness is typically the lower than North American water hardness, typically less than 4, for example 3 grains per gallon mixed $Ca^{2+}$/$Mg^{2+}$.

It has been determined that modification by substitution of an amino acid equivalent to residue position of *Bacillus amyloliquefaciens* subtilisin is important in improving the wash performance of the enzyme. The amino acids substituted, inserted or deleted contemplated by the inventors include, but are not limited to alanine (Ala or A), arginine (Arg or R), aspartic acid (Asp or D), asparagines (Asn or N), cysteine (Cys or C), glutamic acid (Glu or E), glutamine (Gln or Q), glycine (Gly or G), histidine (His or H), isoleucine (Iso or I), leucine (Leu or L), lysine (Lys or K), methionine (Met or M), phenylalanine (Phe or F), proline (Pro or P), serine (Ser or S), threonine (Thr or T), tryptophane (Trp or W), tyrosine (Tyr or Y) and/or valine (Val or V). The subtilisin of the invention may

The subtilisin protease variant of the invention may further comprise at least one additional replaced amino acid at one or more residue positions equivalent to residue positions or selected from the group consisting of 6, 9, 11-12, 19, 25, 37-38, 54-59 68, 71, 89, 111, 115, 120, 121-122, 140, 175, 180, 182, 186, 187, 191, 194, 195, 226 234-238, 241, 260-262, 265, 268, 75, 129, 131, 136, 159, 164, 165, 167, 170, 171, 194, 195, 27, 36, 57, 76, 97, 101, 104, 120, 123, 206, 218, 222, 224, 235, 274, 2, 3, 4, 10, 15, 17, 20, 40, 44, 51, 52, 60, 91, 108, 112, 133, 134, 143, 144, 145, 146, 173, 211, 212, 239, 240, 242, 243, 245, 252, 255, 257, 259, 263, 269, 183, 184, 185, 192, 209, 210, 18, 117, 137, and 244 of *Bacillus amyloliquefaciens.* Specific residues contemplated by the inventors include those equivalent to : I122A, Y195E, M222A, M222S, Y167A, R170S, A194P, D36, N76D, H120D, G195E, and K235N of *Bacillus amyloliquefaciens,* which variant is derived from a *Bacillus* subtilisin. Those skilled in the art will recognize the protease variants having these modifications can be made and are described in US Patents 5,741,694; 6,190,900; and 6,197,567.

The variant may further comprise at least one additional replaced amino acid at one or more equivalent residue positions from the group consisting of 12, 271, 204, 103, 136, 150, 89, 24, 38, 218, 52, 172, 43, 93, 30, 50, 57, 119, 108, 206, 16, 145, 263, 99, 252, 136, 32, 155, 104, 222, 166, 64, 33, 169, 189, 217, 157, 156, 152, 21, 22, 24, 36, 77, 87, 94, 95, 96, 110, 197, 204 107, 170, 171, 172, 213, 67, 135, 97, 126, 127, 128, 129, 214, 215, 50, 124, 123 or 274 of *Bacillus amyloliquefaciens.* Specific residues contemplated by the inventors include: Y217L, K27R, V104Y, N123S, T274A, N76D, S103A, V104I, S101G, S103A, V104I, G159D, A232V, Q236H, Q245R, N248D, N252K M50, M124 and M222S. Additional specific residues contemplated by the inventors include those equivalent to : Q12R, E271G, N204D, S103C, E136G, V150A, E89G, F24S, T38S, N218S, G52E, A172T, N43D, V93I, V30A, L50F, T57A, M119V, A108V, Q206R, I16D, R145G, Y263H, S99G, N252S, Q136R of *Bacillus amyloliquefaciens.* Protease variants, recombinant DNA encoding mutants at these positions and/or methods for making these modifications are described in US patent Nos. RE 34,606; 5,972,682; 5,185,258; 5,310,675; 5,316,941; 5,801,038; 5,972,682, 5,955,340 and 5,700,676. In addition, these modifications can also be made using direct *Bacillus* transformation methods as described in Provisional Application Ser. No. 60/423,087 (filed November 1, 2002; Neelam Amin and Volker Schellenberger). The modifications may be performed using fusion PCR techniques (Teplyakov, AV, et al, Protein Eng., 1992 Jul 5(5):413-20).

These substitutions are preferably made in *Bacillus lentus* (recombinant or native-type) subtilisin, although the substitutions may be made in any *Bacillus* protease.

Based on the screening results obtained with the variant proteases, the noted mutations in *Bacillus amyloliquefaciens* subtilisin and their equivalent in *Bacillus lentus* are important to the proteolytic activity, performance and/or stability of these enzymes and the cleaning or wash performance of such variant enzymes.

Many of the protease variants of the invention are useful in formulating various detergent compositions or personal care formulations such as shampoos or lotions. A number of known compounds are suitable surfactants useful in compositions comprising the protease mutants of the invention. These include nonionic, anionic, cationic, or zwitterionic detergents, as disclosed in US 4,404,128 to Barry J. Anderson and US 4,261,868 to Jiri Flora, et al. A suitable detergent formulation is that described in Example 7 of US Patent 5,204,015. The art is familiar with the different formulations which can be used as cleaning compositions. In addition to typical cleaning compositions, it is readily understood that the protease variants of the present invention may be used for any purpose that native or wild-type proteases are used. Thus, these variants can be used, for example, in bar or liquid soap applications, dishcare formulations, contact lens cleaning solutions or products, peptide hydrolysis, waste treatment, textile applications, as fusion-cleavage enzymes in protein production, etc. The variants of the present invention may comprise enhanced performance in a detergent composition (as compared to the precursor). As used herein, enhanced performance in a detergent is defined as increasing cleaning of certain enzyme sensitive stains such as grass or blood, as determined by usual evaluation after a standard wash cycle.

Proteases of the invention can be formulated into known powdered and liquid detergents having pH between 6.5 and 12.0 at levels of about 0.01 to about 5% (preferably 0.1 % to 0.5%) by weight. These detergent cleaning compositions can also include other enzymes such as known proteases, amylases, cellulases, lipases or endoglycosidases, as well as builders and stabilizers.

The addition of proteases of the invention to conventional cleaning compositions does not create any special use limitation. In other words, any temperature and pH suitable for the detergent is also suitable for the present compositions as long as the pH is within the above range, and the temperature is below the described protease's denaturing temperature. In addition, proteases of the invention can be used in a cleaning composition without detergents, again either alone or in combination with builders and stabilizers.

The present invention also relates to cleaning compositions containing the protease variants of the invention. The cleaning compositions may additionally contain additives which are commonly used in cleaning compositions. These can be selected from, but not limited to, bleaches, surfactants, builders, enzymes and bleach catalysts. It would be readily apparent to one of ordinary skill in the art what additives are suitable for inclusion into the compositions. The list provided herein is by no means exhaustive and should be only taken as examples of suitable additives. It will also be readily apparent to one of ordinary skill in the art to only use those additives which are compatible with the enzymes and other components in the composition, for example, surfactant.

When present, the amount of additive present in the cleaning composition is from about 0.01% to about 99.9%, preferably about 1% to about 95%, more preferably about 1% to about 80%.

The variant proteases of the present invention can be included in animal feed such as part of animal feed additives as described in, for example, US 5,612,055; US 5,314,692; and US 5,147,642, or in

a composition for the treatment of a textile. The composition can be used to treat for example silk or wool as described in publications such as RD 216,034; EP 134,267; US 4,533,359; and EP 344,259.

The following is presented by way of example and is not to be construed as a limitation to the scope of the claims.

Example 1

A large number of protease variants were produced and purified using methods well known in the art. All mutations were made in *Bacillus lentus* GG36 subtilisin. The variants are shown in Table 1.

To construct the GG36 site saturated libraries and site specific variants, three PCR reactions were performed: two PCR's to introduce the mutated codon of interest in GG36 and a fusion PCR to construct the expression vector including the desired mutation(s).

The GG36 codons of interest are numbered according to the BPN' numbering (listed in Figures 1A - C and 3A-B).

For the site saturated library construction:

The method of mutagenesis was based on the region-specific mutation approach (Teplyakov *et al.*, 1992) in which the creation of all possible mutations at a time in a specific DNA codon was performed using a forward and reversed complimentary oligonucleotide primer set with a length of 30-40 nucleotides enclosing a specific designed triple DNA sequence NNS ((A,C,T or G), (A,C,T or G), (C or G)) that correspond with the sequence of the codon to be mutated and guarantees randomly incorporation of nucleotides at that codon.

For the site specific variant construction:

The forward and reverse mutagenic primer enclose the desired mutation(s) in the middle of the primer with ~15 bases of homologues sequence on both sides. These mutation(s), which cover the codon of interest, are specific for the desired amino acid and are synthesized by design.

The second primer set used to construct the libraries and variants contains the pVS08 *Apa*I digestion site together with its flanking nucleotide sequence.

ApaI primers:

Forward *ApaI* primer:

GTGTGT*GGGCCCA*TCAGTCTGACGACC

Reverse *ApaI* primer:

GTGTGT*GGGCCC*TATTCGGATATTGAG

The introduction of the mutation(s) in GG36 molecules was performed using Invitrogen (Carlsbad, CA, USA) Platinum® *Taq* DNA Polymerase High Fidelity (Cat. no. 11304-102) together with pVS08 template DNA and Forward mutagenic primer and Reverse *Apa*I primer for reaction 1, or Reverse mutagenic primer and Forward *Apa*I primer for reaction 2.

The construction of the expression vector including the desired mutation(s) was accomplished by a fusion PCR using PCR fragment of both reaction 1 and 2, forward and reverse *Apa*I primer and Invitrogen Platinum® *Taq* DNA Polymerase High Fidelity (Cat. no. 11304-102).

All PCR's were executed according to Invitrogen protocol supplied with the polymerases, except for the number of cycles: 20 instead of 30. Two separate PCR reactions are performed using Invitrogen Platinum® *Taq* DNA Polymerase High Fidelity (Cat. no. 11304-102):

The amplified linear 5.6 Kb fragment was purified (using Qiagen® Qiaquick PCR purification kit Cat. no. 28106) and digested with *Apa*I restriction enzyme to create cohesive ends on both sides of the fusion fragment:

- 35 μL purified DNA fragment
- 4 μL React® 4 buffer (Invitrogen®: 20 mM Tris-HCl, 5 mM $MgCl_2$, 50 mM KCl, pH 7.4)
- 1 μL *Apa*I*,* 10 units/ml (Invitrogen® Cat. no. 15440-019)

Reaction conditions: 1 hour, 30°C.

An additional digestion with Invitrogen *Dpn*I was performed to remove the pVS08 template DNA:

- 40 μL *Apa*I digested DNA fragment
- 1 μL *Dpn*I*,* 4 units/μL (Invitrogen® Cat. no. 15242-019)

Reaction conditions: 16-20 hours, 37°C.

Ligation of the double digested and purified fragment results in new circular DNA containing the desired mutation with was directly transformed to competent *Bacillus subtilis :*

- 30 μL of purified *Apa*I and *Dpn*I digested DNA fragment
- 8 μL T4 DNA Ligase buffer (Invitrogen® Cat. no. 46300-018)
- 1 μL T4 DNA Ligase, 1 unit/μL (Invitrogen® Cat. no. 15224-017)

Reaction conditions: 16-20 hours, 16°C.

Ligation mixtures were transformed to *Bacillus subtilis* BG2864 (Naki *et al.,* 1998) using the method of Anagnostopoulos and Spizizen (1961) and selected for chloramphenicol resistance and protease activity.

**Method for protein production**

Inoculated 1-50 μL of glycerol culture in Mops media (Frederick C. Neidhardt *et al*., 1974) containing carbon source (Glucose and Maltodextrine, 10.5 and 17.5 g/l) a nitrogen source (Urea, 3.6 g/l), and essential nutrients such as phosphate (0.5 g/l) and sulphate (0.5 g/l) and further supplemented with trace elements (Fe, Mn, Zn, Cu, Co, 1-4 mg/ml). The medium was buffered with a MOPS/Tricine mixture resulting in a pH varying 7 to 8. Incubate the culture for 1-5 days at 37°C/220 rpm (Infors HT® Multitron II).

**References:**

Protein engineering of the high-alkaline serine protease PB92 from Bacillus alcalophilus: functional and structural consequences of mutation at the S4 substrate binding pocket. Teplyakov AV, van der Laan JM, Lammers AA, Kelders H, Kalk KH, Misset O, Mulleners LJ, Dijkstra BW. Protein Eng. 1992 Jul;5(5):413-20.

Selection of a subtilisin-hyperproducing Bacillus in a highly structured environment by D. Naki, C. Paech, G. Ganshaw, V. Schellenberger. Appl Microbiol Biotechnol (1998) 49:290-294.

Requirements for transformation in Bacillus subtilis by Anagnostopoulos, C. and Spizizen, J. in J. Bacteriol. 81, 741-746 (1961).

Culture Medium for Enterobacteria by Frederick C. Neidhardt, Philip L. Bloch and David F. Smith in Journal of Bacteriology, Sept 1974. p736-747 Vol. 119. No. 3.

**Table 1**

| | | | | | | |
|---|---|---|---|---|---|---|
| | | | | | | |
| A1E | | | | | | |
| A1D | | | | | | |
| A1R | | | | | | |
| A1K | | | | | | |
| W6R | | | | | | |
| G7N | | | | | | |
| 18V | | | | | | |
| R10C | | | | | | |
| Q12H | | | | | | |
| G23A | | | | | | |
| F24S | L148G | | | | | |
| G25S | | | | | | |
| V26S | | | | | | |
| V26S | N218S | | | | | |
| V26T | | | | | | |
| E27R | | | | | | |
| V28C | | | | | | |
| V28S | | | | | | |
| A29G | | | | | | |
| V28T | | | | | | |
| V30A | | | | | | |
| L31A | | | | | | |
| L31I | | | | | | |
| L31T | | | | | | |
| L31V | | | | | | |
| R45I | | | | | | |
| T38S | | | | | | |
| G47D | | | | | | |
| G47S | | | | | | |
| S49D | | | | | | |
| S49E | | | | | | |
| D60N | | | | | | |
| G61E | | | | | | |
| G61K | | | | | | |
| G61R | | | | | | |
| G65M | | | | | | |
| T66D | | | | | | |
| T66E | | | | | | |

(continued)

| | | | | | | |
|---|---|---|---|---|---|---|
| G69G | Q12R | | | | | |
| I72C | | | | | | |
| I72L | | | | | | |
| I72V | | | | | | |
| A73L | | | | | | |
| A73G | | | | | | |
| A73T | | | | | | |
| A73V | | | | | | |
| L82R | | | | | | |
| A85D | | | | | | |
| A85G | | | | | | |
| A85L | | | | | | |
| A85S | | | | | | |
| A85V | | | | | | |
| A85Y | | | | | | |
| P86E | | | | | | |
| P86H | E271G | | | | | |
| P86D | | | | | | |
| P86Y | | | | | | |
| A85G | | | | | | |
| A88S | | | | | | |
| L90A | | | | | | |
| L90I | | | | | | |
| L90M | | | | | | |
| L90V | N204D | | | | | |
| A92E | | | | | | |
| A92R | | | | | | |
| V93A | S103C | | | | | |
| V93I | | | | | | |
| V93G | | | | | | |
| V93S | | | | | | |
| V93T | E136G | | | | | |
| K94T | | | | | | |
| K94Q | | | | | | |
| G97C | | | | | | |
| G97E | | | | | | |
| S99C | | | | | | |
| S99D | | | | | | |
| S99G | | | | | | |

(continued)

| | | | | | | |
|---|---|---|---|---|---|---|
| S103D | | | | | | |
| S103E | | | | | | |
| S103T | | | | | | |
| S105D | | | | | | |
| S105E | | | | | | |
| S105G | | | | | | |
| S105R | | | | | | |
| W113D | | | | | | |
| A114C | | | | | | |
| A114G | | | | | | |
| A114S | | | | | | |
| A114T | | | | | | |
| N116D | | | | | | |
| N117S | | | | | | |
| M119A | | | | | | |
| M119C | | | | | | |
| M119F | | | | | | |
| M119G | | | | | | |
| M119S | | | | | | |
| M119T | | | | | | |
| M119V | | | | | | |
| Q121i | | | | | | |
| H120R | | | | | | |
| G127A | | | | | | |
| S128D | | | | | | |
| S128L | | | | | | |
| E136R | | | | | | |
| V139A | V150A | | | | | |
| A142E | E89G | | | | | |
| V147C | | | | | | |
| V147G | | | | | | |
| V147L | | | | | | |
| V147S | | | | | | |
| L148G | | | | | | |
| L148G | F24S | | | | | |
| L148W | | | | | | |
| V149A | | | | | | |
| V149F | | | | | | |
| V149G | | | | | | |

(continued)

| | | | | | | |
|---|---|---|---|---|---|---|
| V149H | | | | | | |
| V149S | Q12H | | | | | |
| V149W | | | | | | |
| V150A | T38S | | | | | |
| V150C | N218S | | | | | |
| V150F | | | | | | |
| V150L | | | | | | |
| A151V | | | | | | |
| S156E | | | | | | |
| S156D | | | | | | |
| A169G | | | | | | |
| R170M | | | | | | |
| A174G | N204D | | | | | |
| A174S | G52E | A172T | | | | |
| A174S | | | | | | |
| A174T | | | | | | |
| G178C | N43D | | | | | |
| G178L | | | | | | |
| G178S | | | | | | |
| I198A | | | | | | |
| I198L | | | | | | |
| I198M | V931 | | | | | |
| 1198V | | | | | | |
| I198V | V30A | | | | | |
| I198T | | | | | | |
| M199V | | | | | | |
| A200S | N204D | | | | | |
| P201C | | | | | | |
| P201S | L50F | | | | | |
| P201S | T57A | | | | | |
| V203R | | | | | | |
| V203D | | | | | | |
| V203E | | | | | | |
| V203L | | | | | | |
| V203S | | | | | | |
| S216D | | | | | | |
| S216E | | | | | | |
| S216R | | | | | | |
| N218S | | | | | | |

(continued)

| | | | | | | |
|---|---|---|---|---|---|---|
| A231G | M119V | | | | | |
| A231 S | | | | | | |
| A232C | | | | | | |
| A231 V | Q206R | | | | | |
| A232G | | | | | | |
| A2321 | A108V | | | | | |
| A232L | | | | | | |
| A232M | | | | | | |
| A231V | Q206R | | | | | |
| A232N | N116D | | | | | |
| A232N | I16D | | | | | |
| A232T | | | | | | |
| A232V | | | | | | |
| A232S | | | | | | |
| L233G | | | | | | |
| L233V | | | | | | |
| I246M | | | | | | |
| I246V | | | | | | |
| R247C | | | | | | |
| S256G | | | | | | |
| T253D | | | | | | |
| T253E | | | | | | |
| T253K | | | | | | |
| T253R | | | | | | |
| G258D | | | | | | |
| G258E | | | | | | |
| G258K | | | | | | |
| G258R | | | | | | |
| G264S | R145G | | | | | |
| L2671 | Y263H | | | | | |
| L267R | | | | | | |
| L267R | S99G | | | | | |
| L267R | N252S | | | | | |
| A270L | | | | | | |
| A270V | E136R | | | | | |
| A273S | | | | | | |
| T260A | | | | | | |

Example 2

A large number of the protease variants produced in Example 1 were tested for performance in two types of detergent and wash conditions using a microswatch assay described in "An improved method of assaying for a preferred enzyme and/or preferred detergent composition", U.S. Serial No. 09/554,992 [WO 99/34011].

Table 2 lists the variant proteases assayed and the results of testing in two different detergents. For column B, the detergent was 7.6 g/l filtered Ariel Regular (Procter & Gamble, Cincinnati, OH, USA), in a solution containing 15 grains per gallon mixed $Ca^{2+}/Mg^{2+}$ hardness, and 0.5 ppm enzyme was used in each well at 40°C [European conditions]. For columns A, the detergent was 0.67 g/l filtered Tide Opal (Procter & Gamble, Cincinnati, OH, USA), in a solution containing 3 grains per gallon mixed $Ca^{2+}/Mg^{2+}$ hardness, and 0.5 ppm enzyme was used in each well at 20°C [Japanese conditions]. A performance index was calculated by the following formula :

Cleaning performance of the variant divided by cleaning performance of GG36 (wild-type)

Four performance values were averaged to arrive at the values shown in Table 2.

**Table 2**

| | | | | A | B | |
|---|---|---|---|---|---|---|
| GG36[1] | | | | 1.00 | 1.00 | |
| GG36- | L31A | | | | 1.4 | |
| GG36- | L82R | | | | 1.2 | |
| GG36- | V203R | | | | 1.6 | |
| GG36- | L233G | | | | 1.2 | |
| GG36- | G258R | | | | 1.6 | |
| GG36- | L267R | | | | 1.2 | |
| GG36- | A270L | | | | 1.3 | |
| GG36- | L311 | | | 1.3 | | |
| GG36- | L31V | | | 1.4 | | |
| GG36- | A85G | | | 1.5 | | |
| GG36- | P86D | | | 1.2 | | |
| GG36- | A92E | | | 1.6 | | |
| GG36- | L148G | | | 1.5 | | |
| GG36- | V149W | | | 1.4 | | |
| GG36- | A151V | | | 1.3 | | |
| GG36- | P201C | | | 1.3 | | |
| GG36- | V203E | | | 1.5 | | |
| GG36- | F24S | L148G | | | 1.2 | |
| GG36- | L50F | P201 S | | | 1.2 | |
| GG36- | S99G | L267R | | | 1.2 | |
| GG36- | T57A | P201 S | | | 1.1 | |
| GG36- | Q206R | A231V | | | 1.3 | |
| GG36- | N252S | L267R | | | 1.2 | |
| GG36- | Q136R | A270V | | | 1.4 | |
| GG36- | L90V | N204D | | 1.4 | | |
| GG36- | A172T | A174S | G52E | 1.1 | | |

(continued)

| | | | | A | B | |
|---|---|---|---|---|---|---|
| GG36- | A174G | N204D | | 1.2 | | |
| GG36- | A200S | N204D | | 1.2 | | |
| GG36- | R145G | G264S | | 1.1 | | |
| [1]GG 36 is the wild type protease of *Bacillus lentus* (SEQ ID NO. 4) | | | | | | |

As a result of the above described assays, some variants exhibited a performance index greater than that of the GG36 wild type protease. For example, the variants L31A, L82R, V203R, L233G, G258R, L267R, and A270L exhibited performance indices of 1.4, 1.2, 1.6, 1.2, 1.6, 1.2, and 1.3 respectively (Column B), in a microswatch assay (WO 99/34011) under European conditions (15 grains per gallon mixed $Ca^{2+}/Mg^{2+}$ hardness, 40 degrees Centigrade, 0.5 ppm). For example, the variants L148G-F24S, P201S-L50F, L267R-S99G, P201S-T57A, A231V-Q206R, L267R-N252S, and A270V-Q136R exhibited performance indices of 1.2, 1.2, 1.2, 1.1, 1.3, 1.2, and 1.4 respectively (Column B), in a microswatch assay (WO 99/34011) under European conditions (15 grains per gallon mixed $Ca^{2+}/Mg^{2+}$ hardness, 40 degrees Centigrade, 0.5 ppm). The variants L311, L31V, A85G, A92E, L148G, V149W, A151V, P201C and V203E exhibited performance indices of 1.3, 1.4, 1.5, 1.2, 1.6, 1.5, 1.4, 1.3, 1.3, and 1.5 respectively (Column A), in the Microswatch 96 microtiter well plate (WO 99/34011) assay under Japanese conditions (3 grains per gallon mixed $Ca^{2+}/Mg^{2+}$ hardness 20 degrees centigrade, 0.5 ppm). The variants N204D-L90V, A174S-A172T-G52E, A174G-N204D, A200S-N204D,R145G-G264S exhibited performance indices of 1.4, 1.1, 1.2, 1.2 and 1.1 respectively (Column A), in the Microswatch 96 microtiter well plate (WO 99/34011) assay under Japanese conditions (3 grains per gallon mixed $Ca^{2+}/Mg^{2+}$ hardness 20 degrees centigrade, 0.5 ppm).

Example 3 Thermostabililty

Thermal stability of protease variants in European detergent solution was examined.

Materials :

iEMS Incubator (Lab systems)
Microtiter plate Reader
ASYS Multispense
Beckman Biomek FX robot
96-well microtiter plate
Ariel Futur detergent (batch'97)
N-Succinyl-Ala-Ala-Pro-Phe p NitroAnilide(AAPF); Sigma S-7388
Tween 80; Sigma P-8074
Tris(hydroxymethyl)aminomethane (Tris); T-1378

Sample preparation:

Enzyme samples where diluted to about 6.0 ppm (protein) starting concentration in 10 mM NaCl/0.005% Tween 80®). A 10 μl of diluted enzyme solution was transferred into 190 μl of unfiltered 3.4 g/L Ariel Futur (Procter & Gamble, Cinncinati, Ohio, USA) with 15 grains per gallon water hardness. The pH was adjusted to 8.6.

Samples were assayed using standard succinyl-ala-ala-pro-phe-para-nitro anilide ("SAAPFpNA") assay (Delmar, E.G., et al Anal. Biochem. 94 (1979) 316-320; Achtstetter, Arch. Biochem. Biophys 207:445-54 (1981)) (pH 8.6, ambient temperature) prior to incubation. For the assay, 10 ul of the sample solution and 200 ul of 1mg/ml SAAPFpNA substrate in 100mMTris pH 8.6 [and 0.005% Tween-80]. After standing at room temperature for thirty minutes after mixing, the absorbance at 405 nm ($OD_{405}$) was determined. The samples were then incubated at 55 C for 20 minutes and the absorbance at 405 nm ($OD_{405}$) was determined. The remaining activity was calculated by dividing the $OD_{405}$ before incubation with the $OD_{405}$ after incubation. Column A depicts the residual activity of the variant divided by the residual activity of the wild-type GG36. For clarification, the mutants were made in GG36, e.g., G7N means the glycine at position 7 was substituted with an asparagines. The results are depicted in Table 3.

**Table 3**

| | | A | | | | | |
|---|---|---|---|---|---|---|---|
| GG36 | | 1.0 | | | | | |
| G7N | | 1.8 | | | | | |
| I8V | | 1.1 | | | | | |
| G23A | | 1.2 | | | | | |
| V26T | | 1.3 | | | | | |
| V28C | | 1.2 | | | | | |
| V28S | | 1.3 | | | | | |
| A29G | | 1.7 | | | | | |
| V30A | | 1.7 | | | | | |
| L31A | | 1.4 | | | | | |
| L31 T | | 1.9 | | | | | |
| G65M | | 1.3 | | | | | |
| N117S | | 1.4 | | | | | |
| I72C | | 1.5 | | | | | |
| I72L | | 1.3 | | | | | |
| I72V | | 1.2 | | | | | |
| A73G | | 1.2 | | | | | |
| A73T | | 1.4 | | | | | |
| A85G | | 1.1 | | | | | |
| A85S | | 1.5 | | | | | |
| A85V | | 1.1 | | | | | |
| P86Y | | 1.3 | | | | | |
| A88S | | 1.5 | | | | | |
| L90A | | 1.2 | | | | | |
| L90I | | 1.4 | | | | | |
| L90M | | 1.5 | | | | | |
| V93G | | 1.2 | | | | | |
| V93S | | 2.5 | | | | | |
| A114C | | 1.1 | | | | | |
| A114G | | 1.2 | | | | | |
| A114S | | 1.2 | | | | | |
| A114T | | 1.2 | | | | | |
| M119A | | 1.5 | | | | | |
| M119C | | 1.1 | | | | | |
| M119F | | 1.4 | | | | | |
| M119G | | 1.2 | | | | | |
| M119Q | | 1.2 | | | | | |

(continued)

| | | A | | | | | |
|---|---|---|---|---|---|---|---|
| M119S | | 1.3 | | | | | |
| M119T | | 1.1 | | | | | |
| M119V | | 1.1 | | | | | |
| M119L | | 1.5 | | | | | |
| V147C | | 1.2 | | | | | |
| V147G | | 1.1 | | | | | |
| V147S | | 1.1 | | | | | |
| V147L | | 1.1 | | | | | |
| L148G | | 1.9 | | | | | |
| V149A | | 1.3 | | | | | |
| V149F | | 1.3 | | | | | |
| V149G | | 1.4 | | | | | |
| V149H | | 1.4 | | | | | |
| V150F | | 1.2 | | | | | |
| V150L | | 1.2 | | | | | |
| V177R | | 1.3 | | | | | |
| G178L | | 2.7 | | | | | |
| G178S | | 2.0 | | | | | |
| I198A | | 1.1 | | | | | |
| I198L | | 1.3 | | | | | |
| I198T | | 1.2 | | | | | |
| I198V | | 1.4 | | | | | |
| V203A | | 1.5 | | | | | |
| V203T | | 1.3 | | | | | |
| A228G | | 1.5 | | | | | |
| A228R | | 1.1 | | | | | |
| A228S | | 1.5 | | | | | |
| A231 S | | 1.3 | | | | | |
| A232C | | 1.3 | | | | | |
| A232G | | 1.2 | | | | | |
| A232L | | 1.2 | | | | | |
| A232M | | 1.3 | | | | | |
| A232S | | 1.2 | | | | | |
| A232T | | 1.2 | | | | | |
| A232V | | 1.2 | | | | | |
| I246M | | 1.3 | | | | | |
| I246V | | 1.2 | | | | | |
| A273S | | 1.1 | | | | | |

(continued)

| | | A | | | | | |
|---|---|---|---|---|---|---|---|
| V26S | N218S | 1.4 | | | | | |
| V93T | E136G | 1.4 | | | | | |
| V139A | V150A | 1.4 | | | | | |
| E89G | A142E | 1.3 | | | | | |
| Q12H | V149S | 1.5 | | | | | |
| V150C | N218S | 1.3 | | | | | |
| T38S | V150A | 1.3 | | | | | |
| N43D | G178C | 2.0 | | | | | |
| V93I | I198M | 1.2 | | | | | |
| V30A | I198V | 1.2 | | | | | |
| M199V | A231 G | 1.1 | | | | | |
| A108V | A2321 | 1.2 | | | | | |
| N116D | A232M | 1.4 | | | | | |
| Y263H | L267I | 1.1 | | | | | |
| V93A | S103C | 1.4 | | | | | |

As a result of the thermostability studies , the variants set forth in Table 3 were found to exhibit thermostability under the above test conditions as compared to the wild-type GG36 protease.

## Claims

1. A variant *Bacillus* subtilisin protease comprising an amino acid sequence having a substitution at a residue position equivalent to residue position 148 of *Bacillus amyloliquefaciens* subtilisin having the sequence shown in Figure 1 B, wherein said variant has improved wash performance and/or thermostability as compared to the precursor subtilisin protease from which the variant is derived.

2. The variant subtilisin protease of claim 1 wherein said substitution is equivalent to the substitution L148G or L148W in *Bacillus lentus* subtilisin having the sequence shown in Figure 3.

3. A variant subtilisin protease according to claim 1 or claim 2 which is a variant of *Bacillus amyloliquefaciens* subtilisin, *Bacillus lentus* subtilisin, or subtilisin 309.

4. A DNA encoding a variant subtilisin protease of any one of claims 1 to 3.

5. An expression vector encoding the DNA of claim 4.

6. A host cell transformed with the expression vector of claim 5.

7. A cleaning composition comprising the variant subtilisin protease of any one of claims 1 to 3.

8. A method of improving the wash performance or thermostability of a precursor *Bacillus* subtilisin protease, comprising making a substitution in said precursor subtilisin protease at a residue position equivalent to residue position 148 of *Bacillus amyloliquefaciens* subtilisin having the sequence shown in Figure 1B to produce a variant subtilisin protease, wherein said variant subtilisin protease has improved wash performance and/or thermostability as compared to said precursor protease.

9. A method according to claim 8, comprising providing a DNA sequence encoding said variant subtilisin protease and expressing said variant subtilisin protease from said DNA sequence.

**10.** A method according to claim 9 comprising providing a precursor DNA sequence encoding said precursor subtilisin protease and modifying said precursor DNA sequence to obtain a variant DNA sequence encoding said variant subtilisin protease.

**11.** A method according to any one of claims 8 to 10, wherein said substitution is equivalent to the substitution L148G or L148W in *Bacillus lentus* subtilisin having the sequence shown in Figure 3.

**12.** A method according to any one of claims 8 to 11 wherein said precursor subtilisin protease is a *Bacillus amyloliquefaciens* subtilisin, *Bacillus lentus* subtilisin, or subtilisin 309.

## Patentansprüche

**1.** *Bacillus* Subtilisin-Proteasevariante umfassend eine Aminosäuresequenz, die eine Substitution an einer Restposition aufweist, die der Restposition 148 von *Bacillus amyloliquefaciens*-Subtilisin äquivalent ist, das die in Figur 1B gezeigte Sequenz aufweist, wobei die Variante eine verbesserte Waschleistung und/oder Wärmestabilität im Vergleich mit der Vorläufer-Subtilisinprotease aufweist, aus der die Variante abgeleitet ist.

**2.** Subtilisin-Proteasevariante nach Anspruch 1, wobei die Substitution der Substitution L148G oder L148W in *Bacillus lentus*-Subtilisin äquivalent ist, das die in Figur 3 gezeigte Sequenz aufweist.

**3.** Subtilisin-Proteasevariante nach Anspruch 1 oder Anspruch 2, die eine Variante von *Bacillus amyloliquefaciens*-Subtilisin, *Bacillus* lentus-Subtilisin oder Subtilisin 309 ist.

**4.** DNA, die für eine Subtilisin-Proteasevariante nach einem der Ansprüche 1 bis 3 kodiert.

**5.** Expressionsvektor, der für die DNA nach Anspruch 4 kodiert.

**6.** Wirtszelle, die mit dem Expressionsvektor nach Anspruch 5 transformiert worden ist.

**7.** Reinigungszusammensetzung umfassend die Subtilisin-Proteasevariante nach einem der Ansprüche 1 bis 3.

**8.** Verfahren zum Verbessern der Waschleistung und/oder Wärmestabilität einer Vorläufer-Bacillus-Subtilisin-Protease, umfassend das Ausführen einer Substitution in der Vorläufer-Subtilisin-Protease an einer Restposition, die der Restposition 148 von *Bacillus amyloliquefaciens*-Subtilisin äquivalent ist, das die in Figur 1B gezeigte Sequenz aufweist, um eine Subtilisin-Proteasevariante herzustellen, wobei die Subtilisin-Proteasevariante eine verbesserte Waschleistung und/oder Wärmestabilität im Vergleich mit der Vorläuferprotease aufweist.

**9.** Verfahren nach Anspruch 8, umfassend das Bereitstellen einer DNA-Sequenz, die für die Subtilisin-Proteasevariante kodiert und Exprimieren der Subtilisin-Proteasevariante aus der DNA-Sequenz.

**10.** Verfahren nach Anspruch 9, umfassend das Bereitstellen einer Vorläufer-DNA-Sequenz, die für die Vorläufer-Subtilisin-Protease kodiert und das Modifizieren der Vorläufer-DNA-Sequenz, um eine DNA-Sequenzvariante zu erhalten, die für die Subtilisin-Proteasevariante kodiert.

**11.** Verfahren nach einem der Ansprüche 8 bis 10, wobei die Substitution der Substitution L148G oder L148W in *Bacillus lentus*-Subtilisin äquivalent ist, das die in Figur 3 gezeigte Sequenz aufweist.

**12.** Verfahren nach einem der Ansprüche 8 bis 11, wobei die Vorläufer-Subtilisin-Protease ein *Bacillus amyloliquefaciens*-Subtilisin, *Bacillus lentus*-Subtilisin oder Subtilisin 309 ist.

## Revendications

**1.** Variant de protéase de subtilisine de *Bacillus* comprenant une séquence d'acides aminés ayant une substitution à une position de résidu équivalente à la position de résidu 148 d'une subtilisine de *Bacillus amyloliquefaciens* possédant la séquence montrée dans la Figure 1B, où ledit variant présente une performance de lavage et/ou une thermostabilité améliorée lorsqu'il est comparé à la protéase de subtilisine précurseur à partir de laquelle le variant est dérivé.

**2.** Variant de protéase de subtilisine selon la revendication 1, dans lequel ladite substitution est équivalente à la substitution L148G ou L148W dans une subtilisine de *Bacillus lentus* possédant la séquence montrée dans la Figure 3.

**3.** Variant de protéase de subtilisine selon la revendication 1 ou la revendication 2, qui est un variant d'une subtilisine de *Bacillus amyloliquefaciens,* d'une subtilisine de *Bacillus lentus* ou d'une subtilisine 309.

**4.** ADN codant pour un variant de protéase de subtilisine selon l'une quelconque des revendications 1 à 3.

**5.** Vecteur d'expression codant pour l'ADN selon la revendication 4.

**6.** Cellule hôte transformée avec le vecteur d'expression selon la revendication 5.

**7.** Composition nettoyante comprenant le variant de protéase de subtilisine selon l'une quelconque des revendications 1 à 3.

**8.** Procédé pour l'amélioration de la performance de lavage et/ou de la thermostabilité d'une protéase de subtilisine de *Bacillus* précurseur, consistant à faire une substitution dans ladite protéase de subtilisine précurseur à une position de résidu équivalente à la position de résidu 148 d'une subtilisine de *Bacillus amyloliquefaciens* possédant la séquence montrée dans la Figure 1B pour produire un variant de protéase de subtilisine, dans lequel ledit variant de protéase de subtilisine présente une performance de lavage et/ou une thermostabilité améliorée lorsqu'il est comparé à ladite protéase précurseur.

**9.** Procédé selon la revendication 8, comprenant la fourniture d'une séquence d'ADN codant pour ledit variant de protéase de subtilisine et l'expression dudit variant de protéase de subtilisine à partir de ladite séquence d'ADN.

**10.** Procédé selon la revendication 9, comprenant la fourniture d'une séquence d'ADN précurseur codant pour ladite protéase de subtilisine précurseur et la modification de ladite séquence d'ADN précurseur pour obtenir un variant de séquence d'ADN codant pour ledit variant de protéase de subtilisine.

**11.** Procédé selon l'une quelconque des revendications 8 à 10, dans lequel ladite substitution est équivalente à la substitution L148G ou L148W dans une subtilisine de *Bacillus lentus* possédant la séquence montrée dans la Figure 3.

**12.** Procédé selon l'une quelconque des revendications 8 à 11, dans lequel ladite protéase de subtilisine précurseur est une subtilisine de *Bacillus amyloliquefaciens,* une subtilisine de *Bacillus lentus* ou une subtilisine 309.

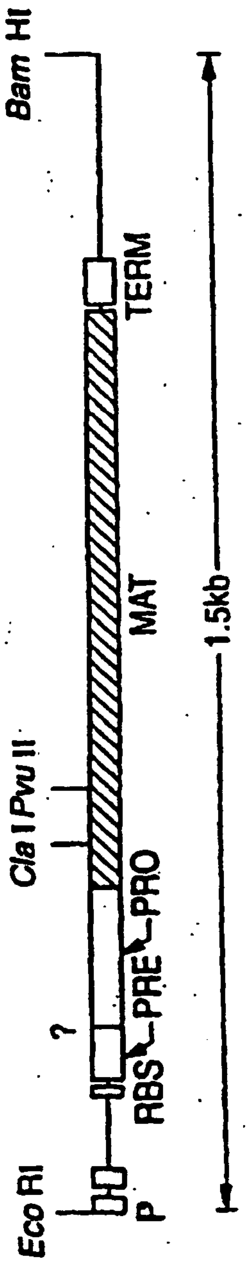


FIG._1A

```
  (5)                      (3)                       (4)                                                  -107
   P                                                                                              -RBS     Met
1  GGTCTACTAAAATATTATTCCATACTATACAATTAATACACAGAATAATCTGTCTATTGGTTATTCTGCAAATGAAAAAAAGGAGAGGATAAAGA GTG

                                -100    PRE                         -90
    Arg Gly Lys Lys Val Trp Ile Ser Leu Leu Phe Ala Leu Ala Leu Ile Phe Thr Met Ala Phe Gly Ser Thr Ser
99  AGA GGC AAA AAA GTA TGG ATC AGT TTG CTG TTT GCT TTA GCG TTA ATC TTT ACG ATG GCG TTC GGC AGC ACA TCC

        -80                                 -70     PRO                                 -60
    Ser Ala Gln Ala Ala Gly Lys Ser Asn Gly Glu Lys Lys Tyr Ile Val Gly Phe Lys Gln Thr Met Ser Thr Met
174 TCT GCC CAG GCG GCA GGG AAA TCA AAC GGG GAA AAG AAA TAT ATT GTC GGG TTT AAA CAG ACA ATG AGC ACG ATG

                            -50                                 -40
    Ser Ala Ala Lys Lys Lys Asp Val Ile Ser Glu Lys Gly Gly Lys Val Gln Lys Gln Phe Lys Tyr Val Asp Ala
249 AGC GCC GCT AAG AAG AAA GAT GTC ATT TCT GAA AAA GGC GGG AAA GTG CAA AAG CAA TTC AAA TAT GTA GAC GCA

        -30                                 -20                                 -10
    Ala Ser Ala Thr Leu Asn Glu Lys Ala Val Lys Glu Leu Lys Lys Asp Pro Ser Val Ala Tyr Val Glu Glu Asp
324 GCT TCA GCT ACA TTA AAC GAA AAA GCT GTA AAA GAA TTG AAA AAA GAC CCG AGC GTC GCT TAC GTT GAA GAA GAT

                        -1 |-> MAT
                           1                                10
    His Val Ala His Ala Tyr Ala Gln Ser Val Pro Tyr Gly Val Ser Gln Ile Lys Ala Pro Ala Leu His Ser Gln
399 CAC GTA GCA CAT GCG TAC GCG CAG TCC GTG CCT TAC GGC GTA TCA CAA ATT AAA GCC CCT GCT CTG CAC TCT CAA

    20                                      30                                      40
    Gly Tyr Thr Gly Ser Asn Val Lys Val Ala Val Ile Asp Ser Gly Ile Asp Ser Ser His Pro Asp Leu Lys Val
474 GGC TAC ACT GGA TCA AAT GTT AAA GTA GCG GTT ATC GAC AGC GGT ATC GAT TCT TCT CAT CCT GAT TTA AAG GTA
```

FIG._1B - 1

```
                                   50                                          Pro Asn         60  Asp
     Ala Gly Gly Ala Ser Met Val Pro Ser Glu Thr Asn Pro Phe Gln Asp Asn Asn Ser His Gly Thr His Val Ala
549  GCA GGC GGA GCC AGC ATG GTT CCT TCT GAA ACA AAT CCT TTC CAA GAC AAC AAC TCT CAC GGA ACT CAC GTT GCC

     70                                      80                                  Ser Ala  90
     Gly Thr Val Ala Ala Leu Asn Asn Ser Ile Gly Val Leu Gly Val Ala Pro Ser Ala Ser Leu Tyr Ala Val Lys
624  GGC ACA GTT GCG GCT CTT AAT AAC TCA ATC GGT GTA TTA GGC GTT GCG CCA AGC GCA TCA CTT TAC GCT GTA AAA

                 Asp Ala 100                                  110
     Val Leu Gly Ala Asp Gly Ser Gly Gln Tyr Ser Trp Ile Ile Asn Gly Ile Glu Trp Ala Ile Ala Asn Asn Met
699  GTT CTC GGT GCT GAC GGT TCC GGC CAA TAC AGC TGG ATC ATT AAC GGA ATC GAG TGG GCG ATC GCA AAC AAT ATG

     120                                     130                                     140
     Asp Val Ile Asn Met Ser Leu Gly Gly Pro Ser Gly Ser Ala Ala Leu Lys Ala Ala Val Asp Lys Ala Val Ala
774  GAC GTT ATT AAC ATG AGC CTC GGC GGA CCT TCT GGT TCT GCT GCT TTA AAA GCG GCA GTT GAT AAA GCC GTT GCA

                         150                             Ser Thr 160
     Ser Gly Val Val Val Val Ala Ala Ala Gly Asn Glu Gly Thr Ser Gly Ser Ser Ser Thr Val Gly Tyr Pro Gly
849  TCC GGC GTC GTA GTC GTT GCG GCA GCC GGT AAC GAA GGC ACT TCC GGC AGC TCA AGC ACA GTG GGC TAC CCT GGT

     170                                     180                                 190
     Lys Tyr Pro Ser Val Ile Ala Val Gly Ala Val Asp Ser Ser Asn Gln Arg Ala Ser Phe Ser Ser Val Gly Pro
924  AAA TAC CCT TCT GTC ATT GCA GTA GGC GCT GTT GAC AGC AGC AAC CAA AGA GCA TCT TTC TCA AGC GTA GGA CCT

                         200                                 210
     Glu Leu Asp Val Met Ala Pro Gly Val Ser Ile Gln Ser Thr Leu Pro Gly Asn Lys Tyr Gly Ala Tyr Asn Gly
999  GAG CTT GAT GTC ATG GCA CCT GGC GTA TCT ATC CAA AGC ACG CTT CCT GGA AAC AAA TAC GGG GCG TAC AAC GGT

     220                                     230                                 240
     Thr Ser Met Ala Ser Pro His Val Ala Gly Ala Ala Ala Leu Ile Leu Ser Lys His Pro Asn Trp Thr Asn Thr
1074 ACG TCA ATG GCA TCT CCG CAC GTT GCC GGA GCG GCT GCT TTG ATT CTT TCT AAG CAC CCG AAC TGG ACA AAC ACT
```

FIG._1B - 2

```
                         250  Gln                                         260
     Gln  Val  Arg  Ser  Ser  Leu  Glu  Asn  Thr  Thr  Thr  Lys  Leu  Gly  Asp  Ser  Phe  Tyr  Tyr  Gly  Lys  Gly  Leu  Ile  Asn
1149 CAA  GTC  CGC  AGC  AGT  TTA  GAA  AAC  ACC  ACT  ACA  AAA  CTT  GGT  GAT  TCT  TTC  TAC  TAT  GGA  AAA  GGG  CTG  ATC  AAC

     270                 275
     Val  Gln  Ala  Ala  Ala  Gln  OC                  TERM
1224 GTA  CAG  GCG  GCA  GCT  CAG  TAA  AACATAAAAAACCGGCCTTGGCCCCGCCGGTTTTTTATTTTTCTTCCTCCGCATGTTCAATCCGCTCC

1316 ATAATCGACGGATGGCTCCCTCTGAAAATTTTAACGAGAAACGGCGGGTTGACCCGGCTCAGTCCCGTAACGGCCAAGTCCTGAAACGTCTCAATCGCCG

1416 CTTCCCGGTTTCCGGTCAGCTCAATGCCGTAACGGTCGGCGGCGTTTTCCTGATACCGGGAGACGGCATTCGTAATCGGATC
```

FIG._1B - 3

FIG._1B - 1

FIG._1B - 2

FIG._1B - 3

FIG._1B

CONSERVED RESIDUES IN SUBTILISINS FROM
*BACILLUS AMYLOLIQUEFACIENS*

```
1                  10                  20
A Q S V P . G . . . . . A P A . H . . G

21                 30                  40
. T G S . V K V A V . D . G . . . . H P

41                 50                  60
D L . . . G G A S . V P . . . . . . Q D

61                 70                  80
. N . H G T H V A G T . A A L N N S I G

81                 90                 100
V L G V A P S A . L Y A V K V L G A . G

101               110                 120
S G . . S . L . . G . E W A . N . . . .

121               130                 140
V . N . S L G . P S . S . . . . . A . .

141               150                 160
. . . . . G V . V V A A . G N . G . . .

161               170                 180
. . . . . . Y P . . Y . . . . A V G A .

181               190                 200
D . . N . . A S F S . . G . . L D . . A

201               210                 220
P G V . . Q S T . P G . . Y . . . N G T

221               230                 240
S M A . P H V A G A A A L . . . K . . .

241               250                 260
W . . . Q . R . . L . N T . . . L G . .

261               270
. . Y G . G L . N . . A A . .
```

*FIG._2*

COMPARISON OF SUBTILISIN SEQUENCES FROM:

*B.amyloliquefaciens*
*B.subtilis*
*B.licheniformis*
*B.lentus*

01 10 20 30
A Q S V P Y G V S Q I K A P A L H S Q G Y T G S N V K V A V I D S G I D S S H P
A Q S V P Y G I S Q I K A P A L H S Q G Y T G S N V K V A V I D S G I D S S H P
A Q T V P Y G I P L I K A D K V Q A Q G F K G A N V K V A V L D T G I Q A S H P
A Q S V P W G I S R V Q A P A A H N R G L T G S G V K V A V L D T G I S T * H P

41 50 60 70
D L K V A G G A S M V P S E T N P F Q D N N S H G T H V A G T V A A L N N S I G
D L N V R G G A S F V P S E T N P Y Q D G S S H G T H V A G T I A A L N N S I G
D L N V V G G A S F V A G E A Y N * T D G N G H G T H V A G T V A A L D N T T G
D L N I R G G A S F V P G E * P S T Q D G N G H G T H V A G T I A A L N N S I G

81 90 100 110
V L G V A P S A S L Y A V K V L G A D G S G Q Y S W I I N G I E W A I A N N M D
V L G V S P S A S L Y A V K V L D S T G S G Q Y S W I I N G I E W A I S N N M D
L G V A P S V S L Y A V K V L N S S G S G S Y S G I V S G I E W A T T N G M D
V L G V A P S A E L Y A V K V L G A S G S G S V S S I A Q G L E W A G N N G M H

121 130 140 150
V I N M S L G G P S G S A A L K A A V D K A V A S G V V V V A A A G N E G T S G
V I N M S L G G P T G S T A L K T V V D K A V S S G I V V A A A A G N E G S S G
V I N M S L G G A S G S T A M K Q A V D N A Y A R G V V V V A A A G N S G N S G
V A N L S L G S P S P S A T L E Q A V N S A T S R G V L V V A A S G N S G A G S

FIG._3A

```
161                 170                 180                 190
S S S T V G Y P G K Y P S V I A V G A V D S S N Q R A S F S S V G P E L D V M A
S T S T V G Y P A K Y P S T I A V G A V N S S N Q R A S F S S A G S E L D V M A
S T N T I G Y P A K Y D S V I A V G A V D S N S N R A S F S S V G A E L E V M A
* * * * I S Y P A R Y A N A M A V G A T D Q N N N R A S F S Q Y G A G L D I V A

201                 210                 220                 230
P G V S I Q S T L P G N K Y G A Y N G T S M A S P H V A G A A A L I L S K H P N
P G V S I Q S T L P G G T Y G A Y N G T S M A T P H V A G A A A L I L S K H P T
P G A G V Y S T Y P T N T Y A T L N G T S M A S P H V A G A A A L I L S K H P N
P G V N V Q S T Y P G S T Y A S L N G T S M A T P H V A G A A A L V K Q K N P S

241                 250                 260                 270
W T N T Q V R S S L E N T T T K L G D S F Y Y G K G L I N V Q A A A Q
W T N A Q V R D R L E S T A T Y L G N S F Y Y G K G L I N V Q A A A Q
L S A S Q V R N R L S S T A T Y L G S S F Y Y G K G L I N V E A A A Q
W S N V Q I R N H L K N T A T S L G S T N L Y G S G L V N A E A A T R
```

FIG._3B

FIG._3

| FIG._3A |
|---|
| FIG._3B |

FIG.- 4

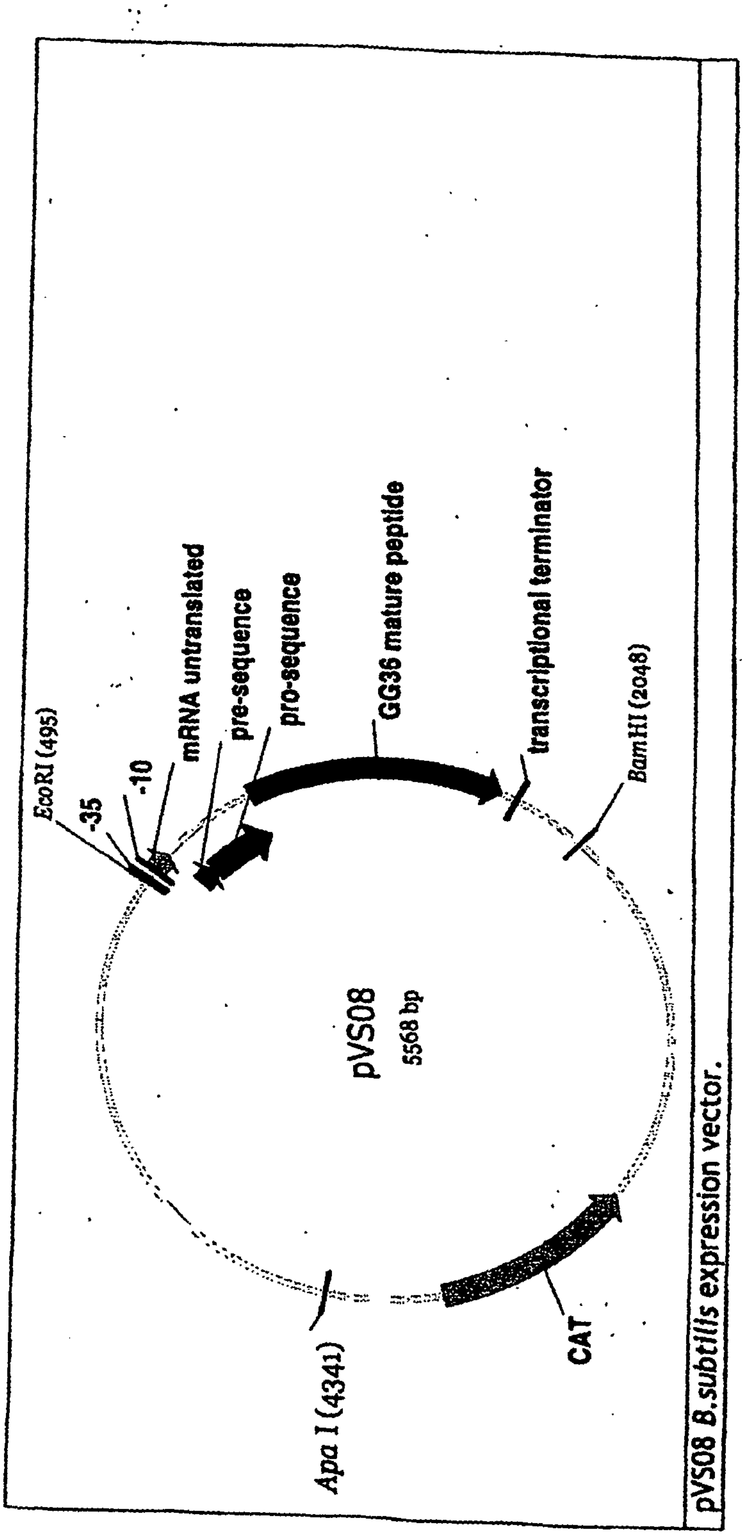


pVS08 *B.subtilis* expression vector.

FIG.- 5

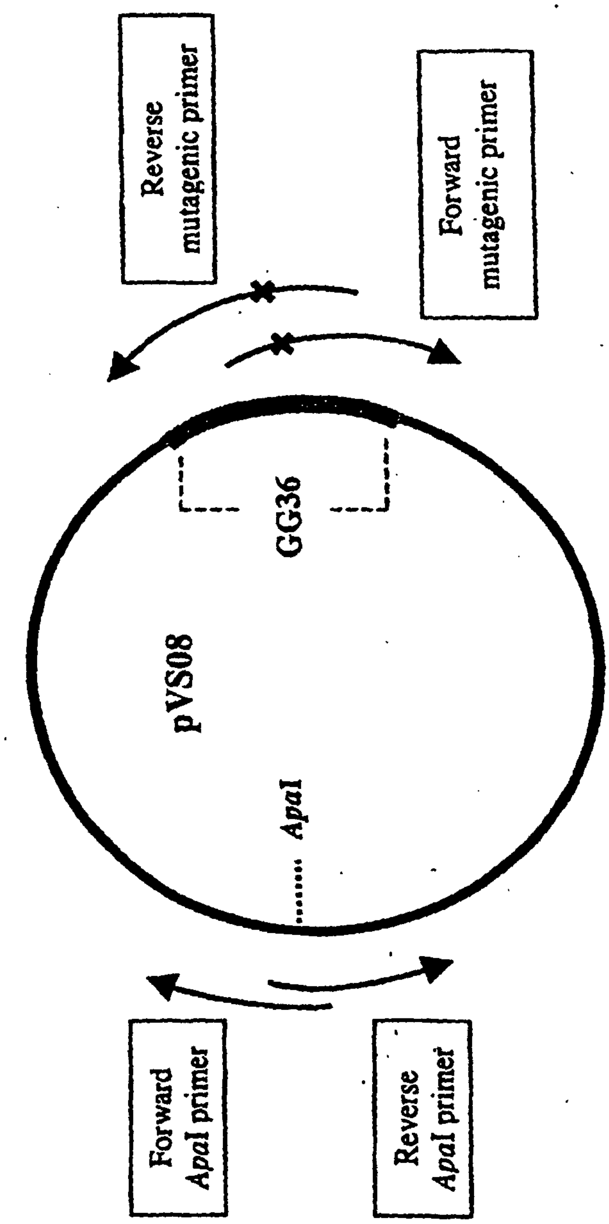
Reverse
mutagenic primer
Forward
mutagenic primer
GG36
pVS08
ApaI
Forward
ApaI primer
Reverse
ApaI primer

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 8906276 A
- US RE34606 E
- US 5204015 A
- US 5185258 A
- US 5700676 A
- US 5801038 A
- US 5763257 A
- EP 0328299 A
- WO 8906279 A
- EP 0251446 A
- US 5182204 A
- US 5441882 A
- US 5264366 A
- US 5741694 A
- US 6190900 A
- US 6197567 A
- US 5972682 E
- US 5185258 E
- US 5310675 E
- US 5316941 E
- US 5801038 E
- US 5955340 E
- US 5700676 E
- US 60423087 B, Neelam Amin and Volker Schellenberger
- US 4404128 A, Barry J. Anderson
- US 4261868 A, Jiri Flora
- US 5612055 A
- US 5314692 A
- US 5147642 A
- WO 216034 A
- EP 134267 A
- US 4533359 A
- EP 344259 A
- US 09554992 B
- WO 9934011 A


### Non-patent literature cited in the description


- **STROUD, R.** *Sci. Amer.,* vol. 131, 74-88
- **KRAUT, J.** *Annu. Rev. Biochem.,* 1977, vol. 46, 331-358
- **MARKLAND, F.S. et al.** *Hoppe-Seyler's Z. Physiol. Chem.,* 1983, vol. 364, 1537-1540
- **ROBERTUS, J.D. et al.** *Biochemistry,* 1972, vol. 11, 2439-2449
- **ROBERTUS, J.D. et al.** *J. Biol. Chem.,* vol. 251, 1097-1103
- **SVENDSEN, B.** *Carlsberg Res. Commun.,* 1976, vol. 41, 237-291
- **STAUFFER, D.C. et al.** *J. Biol. Chem.,* 1965, vol. 244, 5333-5338
- **WELLS ; ESTELL.** *TIBS,* 1988, vol. 13, 291-297
- **DALE, M.W.** Molecular Genetics of Bacteria. John Wiley & Sons, Ltd, 1989
- **SIEZEN et al.** *Protein Eng.,* 1991, vol. 4 (7), 719-737
- **D. NAKI ; C. PAECH ; G. GANSHAW ; V. SCHELLENBERGER.** *Appl Microbiol Biotechnol,* 1988, vol. 49, 290-294
- Microbial Proteinases. **K. M. KALISZ.** Advances in Biochemical Engineering/Biotechnology. 1988
- **TEPLYAKOV, AV et al.** *Protein Eng.,* 05 July 1992, 413-20
- **TEPLYAKOV AV ; VAN DER LAAN JM ; LAMMERS AA ; KELDERS H ; KALK KH ; MISSET O ; MULLENERS LJ ; DIJKSTRA BW.** Protein engineering of the high-alkaline serine protease PB92 from Bacillus alcalophilus: functional and structural consequences of mutation at the S4 substrate binding pocket. *Protein Eng.,* July 1992, vol. 5 (5), 413-20
- **D. NAKI ; C. PAECH ; G. GANSHAW ; V. SCHELLENBERGER.** Selection of a subtilisin-hyperproducing Bacillus in a highly structured environment. *Appl Microbiol Biotechnol,* 1998, vol. 49, 290-294
- **ANAGNOSTOPOULOS, C. ; SPIZIZEN, J.** Requirements for transformation in Bacillus subtilis. *J. Bacteriol.,* 1961, vol. 81, 741-746
- **FREDERICK C. NEIDHARDT ; PHILIP L. BLOCH ; DAVID F. SMITH.** Culture Medium for Enterobacteria. *Journal of Bacteriology,* September 1974, vol. 119 (3), 736-747
- **DELMAR, E.G. et al.** *Anal. Biochem.,* 1979, vol. 94, 316-320
- **ACHTSTETTER.** *Arch. Biochem. Biophys,* 1981, vol. 207, 445-54